# EUROPEAN PATENT APPLICATION

(11) **EP 3 919 472 A1**
(43) Date of publication of application: **08.12.2021**
(21) Application number: 20749066.5
(22) Date of filing: 31.01.2020
(51) Int. Cl.: C07C 215/64, C07C 217/94, C07C 217/58, A61K 31/135, A61P 25/04

(54) **DEZOCINE DERIVATIVE AND MEDICAL USE THEREOF**

(30) Priority: 02.02.2019 CN 201910106523
(71) Applicant: Yangtze River Pharmaceutical Group Co., Ltd., Taizhou, Jiangsu 225321 (CN)
(72) Inventor: SHAO, Liming, Shanghai 201203 (CN); HU, Tao, Taizhou, Jiangsu 225321 (CN); XU, Haoyu, Taizhou, Jiangsu 225321 (CN); CHEN, Lingwu, Taizhou, Jiangsu 225321 (CN); ZOU, Yiquan, Taizhou, Jiangsu 225321 (CN); CAI, Wei, Taizhou, Jiangsu 225611 (CN); YANG, Xicheng, Shanghai 201203 (CN); LI, Haodong, Taizhou, Jiangsu 225321 (CN); XUE, Dengqi, Shanghai 201203 (CN)
(74) Representative: Habermann, Hruschka & Schnabel
(86) International application number: PCT/CN2020/074131
(87) International publication number: WO 2020/156522

(57) **Abstract**

Provided are a dezocine derivative represented by Formula I, or a tautomer, optical isomer, nitrogen oxide, solvate, pharmaceutically acceptable salt or prodrug thereof, as well as a pharmaceutical composition containing the same, preparations thereof, and medical use thereof, and the structure of Formula I is as below:

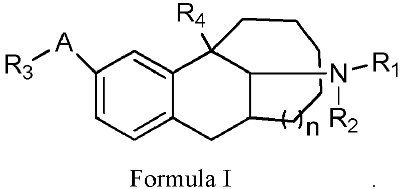

## Description

This application claims priority to an earlier Chinese Patent Application No. 201910106523.7, filed with the China National Intellectual Property Administration on February 2, 2019, titled with "DEZOCINE DERIVATIVE AND MEDICAL USE THEREOF". The disclosure of the aforementioned application is hereby incorporated by reference in its entirety.

### FIELD

The present disclosure relates to the technical field of medicine, and in particular, to a series of new dazocine derivatives, pharmaceutical compositions containing the derivatives, preparation methods thereof, and medical uses thereof.

### BACKGROUND

Dezocine is a mixed agonist-antagonist of opioid receptors with a structure similar to pentazocine. Dezocine was developed by Wyeth-Ayerst Laboratories in the United States in the 1970s and was approved by the FDA in 1989 to be a commercial product, marked by AstraZeneca with the trade name Dalgan for the treatment of postoperative pain. Since its launch in China in 2009, dezocine has been widely used in general anesthesia induction, postoperative analgesia and preemptive analgesia, and for the treatment of visceral pain and cancer pain. Its chemical name is: (-)-[5R-(5α,11α,13S^{∗})]-13-amino-5,6,7,8,9,10,11,12-octahydro-5-methyl-5,11-methylene benzocyclodecen-3-ol, having the structural formula below:

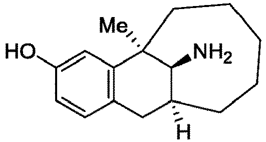

### SUMMARY

The present disclosure provides a compound represented by Formula I, or a tautomer, optical isomer, nitrogen oxide, solvate, pharmaceutically acceptable salt or prodrug thereof: 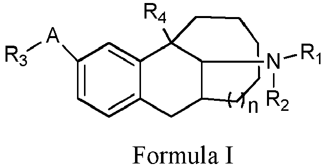
where R₁ and R₂ are each independently selected from H, deuterium, tritium, C₁-C₁₂ aliphatic hydrocarbyl, C₆-C₁₄ aryl, C₆-C₁₄ aryl-C₁-C₁₂ aliphatic hydrocarbyl, C₃-C₈ cycloalkyl, C₃-C₈ cycloalkyl-C₁-C₁₂ aliphatic hydrocarbyl, five- to fourteen-membered heteroaryl, or five-to fourteen-membered heteroaryl-C₁-C₁₂ aliphatic hydrocarbyl, wherein the C₆-C₁₄ aryl, the C₃-C₈ cycloalkyl, and the five- to fourteen-membered heteroaryl are optionally substituted by one or more halogens, -OH groups, C1-C12 aliphatic hydrocarbyl groups, C₁-C₁₂ aliphatic hydrocarbyl oxyl groups, or C₁-C₁₂ aliphatic hydrocarbyl-S- groups; or wherein R₁, R₂ and N connected thereto together form a N-containing four- to six-membered ring, and the N-containing four- to six-membered ring is optionally substituted by one or more halogens, -OH groups, C1-C12 aliphatic hydrocarbyl groups, C₁-C₁₂ aliphatic hydrocarbyl oxyl groups, or C₁-C₁₂ aliphatic hydrocarbyl-S- groups;
R₃ is selected from H, deuterium, tritium C₁-C₁₂ aliphatic hydrocarbyl, C₆-C₁₄ aryl, C₆-C₁₄ aryl-C₁-C₁₂ aliphatic hydrocarbyl, C₃-C₈ cycloalkyl, C₃-C₈ cycloalkyl-C₁-C₁₂ aliphatic hydrocarbyl, five- to fourteen-membered heteroaryl, or five- to fourteen-membered heteroaryl-C₁-C₁₂ aliphatic hydrocarbyl, wherein the C₆-C₁₄ aryl, the C₃-C₈ cycloalkyl, and the five- to fourteen-membered heteroaryl are optionally substituted by one or more halogens, C₁-C₁₂ aliphatic hydrocarbyl groups, C₁-C₁₂ aliphatic hydrocarbyl oxyl groups, or C₁-C₁₂ aliphatic hydrocarbyl-S- groups;
R₄ is selected from H, deuterium, tritium, OH, halogen, C₁-C₁₂ aliphatic hydrocarbyl, C₁-C₁₂ aliphatic hydrocarbyl oxyl, or C₁-C₁₂ aliphatic hydrocarbyl-S-;
at least one of R₁, R₂, or R₃ is not H;
A is selected from O or S; and
n is selected from 0, 1, or 2.

In some embodiments, R₁ and R₂ are each independently selected from H, deuterium, tritium, C₁-C₁₂ aliphatic hydrocarbyl, C₆-C₁₄ aryl-C₁-C₆ aliphatic hydrocarbyl, C₃-C₈ cycloalkyl, or C₃-C₈ cycloalkyl-C₁-C₆ aliphatic hydrocarbyl, wherein the C₆-C₁₄ aryl and the C₃-C₈ cycloalkyl are optionally substituted by one or more halogens, -OH groups, C₁-C₆ aliphatic hydrocarbyl groups, C₁-C₆ aliphatic hydrocarbyl oxyl groups, or C₁-C₆ aliphatic hydrocarbyl-S- groups; or, R₁, R₂ and N connected thereto together form a N-containing four- to six-membered ring;
R₃ is selected from H, deuterium, tritium C₁-C₆ aliphatic hydrocarbyl, or C₆-C₁₄ aryl-Ci-C₁₂ aliphatic hydrocarbyl, wherein the C₆-C₁₄ aryl is optionally substituted by one or more halogens, C₁-C₆ aliphatic hydrocarbyl groups, C₁-C₆ aliphatic hydrocarbyl oxyl groups, or C₁-C₆ aliphatic hydrocarbyl-S- groups;
R₄ is selected from C₁-C₆ aliphatic hydrocarbyl, C₁-C₆ aliphatic hydrocarbyl oxyl, or C₁-C₆ aliphatic hydrocarbyl-S-;
at least one of R₁, R₂, or R₃ is not H;
A is selected from O or S; and
n is selected from 1 or 2.

In some embodiments, R₁ and R₂ are each independently selected from H, deuterium, tritium, C₁-C₁₂ alkyl, C₆-C₁₄ aryl-C₁-C₆ alkyl, C₃-C₈ cycloalkyl, or C₃-C₈ cycloalkyl-C₁-C₆ alkyl, wherein the C₆-C₁₄ aryl and the C₃-C₈ cycloalkyl are optionally substituted by one or more halogens, C₁-C₆ alkyl groups, C₁-C₆ alkoxyl groups, or C₁-C₆ alkyl-S- groups; or, R₁, R₂ and N connected thereto together form a N-containing five-membered ring;
R₃ is selected from H, deuterium, tritium, C₁-C₆ alkyl, or C₆-C₁₄ aryl-C₁-C₁₂ alkyl, wherein the C₆-C₁₄ aryl is optionally substituted by one or more halogens, C₁-C₆ alkyl groups, C₁-C₆ alkoxyl groups, or one or more C₁-C₆ alkyl-S- groups;
R₄ is selected from C₁-C₆ alkyl, C₁-C₆ alkoxyl, or C₁-C₆ alkyl-S-;
at least one of R₁, R₂, or R₃ is not H;
A is O; and
n is 1.

In some embodiments, R₁ is selected from hydrogen, deuterium, tritium, or C₁-C₆ alkyl; R₂ is selected from hydrogen, deuterium, tritium, C₁-C₆ alkyl, or C₆-C₁₄ aryl-C₁-C₆ alkyl; R₃ is selected from H, deuterium, tritium, or C₆-C₁₄ aryl-C₁-C₁₂ alkyl, wherein the C₆-C₁₄ aryl is optionally substituted by one or more halogens, C₁-C₆ alkyl groups, C₁-C₆ alkoxyl groups, or C₁-C₆ alkyl-S- groups. In some embodiments, the C₁-C₁₂ aliphatic hydrocarbyl is selected from methyl, ethyl, n-propyl, isopropyl, n-butyl, isobutyl, tert-butyl, n-pentyl, isopentenyl, neopentyl, n-hexyl, vinyl, 1-propenyl, 2-propenyl, 1-methylvinyl, 1-butenyl, 1-ethylvinyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, or 1-hexynyl; the halogen is selected from F, Cl, Br, or I; the aryl is selected from phenyl or naphthyl; the C3-C8 cycloalkyl is selected from cyclopropyl, cyclobutyl, cyclopentyl, or cyclohexyl; the N-containing four- to six-membered ring is selected from ethylenimine, pyrrolidine, piperidine, piperazine, morpholine, pyrrole, imidazole, pyrazole, thiazole, isothiazole, oxazole, isoxazole, pyridine, pyrazine, pyrimidine, or pyridazine.

In some embodiments, the pharmaceutically acceptable salt is selected from hydrochlorides.

In some embodiments, a structure of Formula I has a structure of Formula II below:

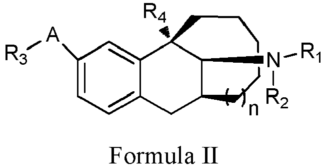

Preferably, the compound represented by Formula (I) is selected from the following compounds:

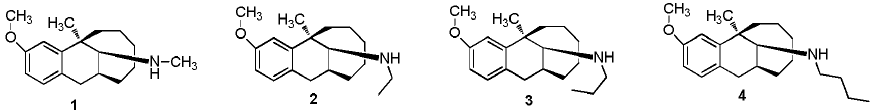

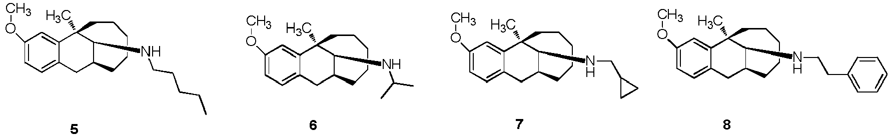

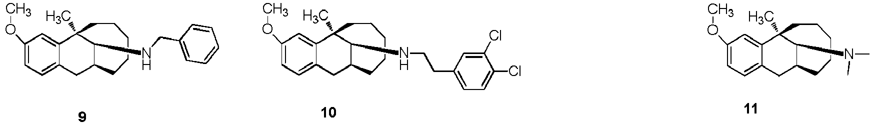

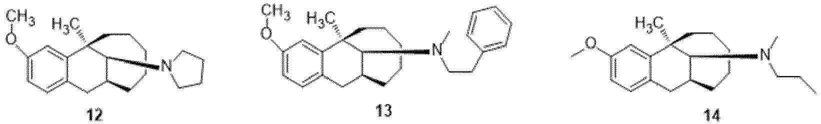

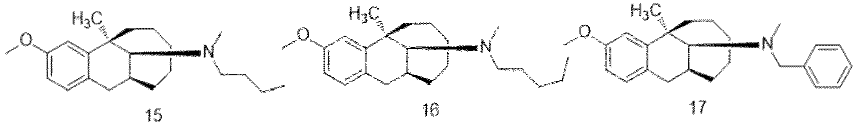

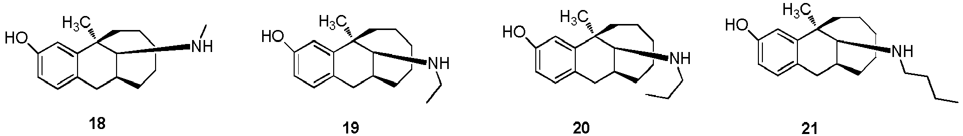

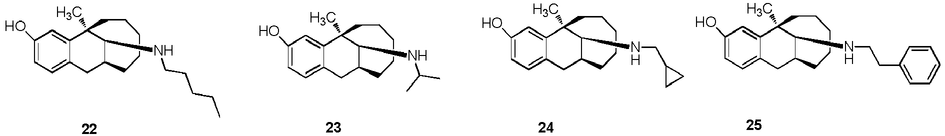

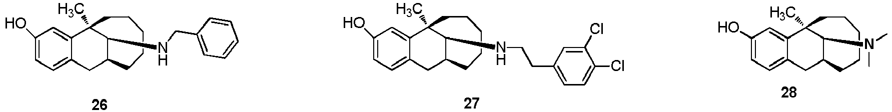

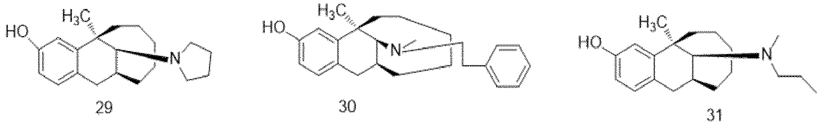

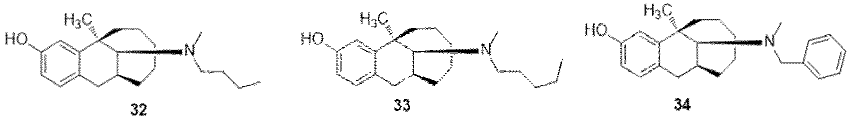

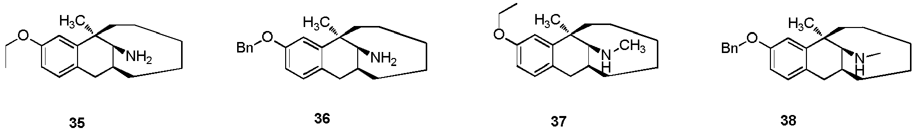

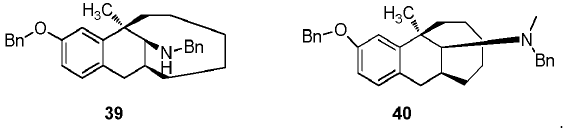

More preferably, the compound represented by Formula (I) is selected from compounds 1-11, 13-36, and 38-40.

Further preferably, the compound represented by Formula (I) is selected from compounds 3, 4, 8, 18-34, 36, and 38-40.

Particularly preferably, the compound represented by Formula (I) is selected from compounds 19-22, 25, 12, 28, 30-34, 39, and 40.

In particular, the compound represented by Formula (I) is selected from compounds 21, 22, 25, 26, 30, 33-34, and 40.

Among the particularly preferred compounds and the most preferred compounds, IC₅₀ values for at least one of µ, κ and δ opioid receptors can reach Grade A; more preferably, IC₅₀ values for at least two of the opioid receptors can reach A; and most preferably, IC₅₀ values for all of the three opioid receptors can reach Grade A.

The present disclosure further provides a preparation method of a compound represented by Formula I (including compound represented by Formula II). The preparation method includes the following synthesis schemes. 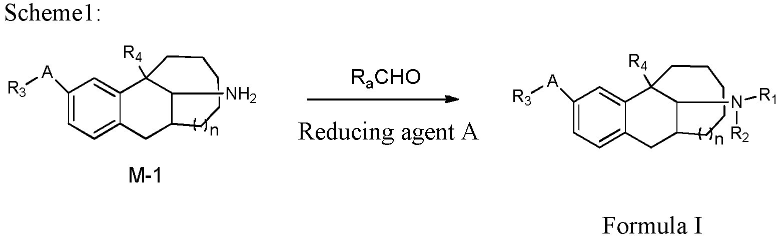 the scheme 1 including:
(1) compound M-1 reacting with an aldehyde RₐCHO to obtain an intermediate T-1; and
(2) obtaining the compound represented by Formula I through a reduction of the intermediate T-1,
in the scheme 1, R₁, R₂, R₃, R₄, A, and n are those as defined in the Formula I, except that at least one of R₁ or R₂ is not H; RₐCHO is an aldehyde compound corresponding to substitutes R₁ and R₂ to be introduced in the target compound represented by Formula I, and allows a mono-substitution or di-substitution reaction with N, wherein when neither of R₁ nor R₂ is H, a disubstituted product is obtained correspondingly; and when one of R₁ and R₂ is H, a monosubstituted product is obtained correspondingly. 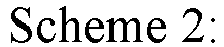 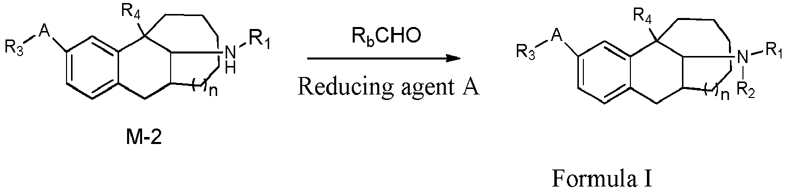 the scheme 2 including:
(1) compound M-2 reacting with an aldehyde R_{b}CHO to obtain an intermediate T-2; and
(2) obtaining the compound represented by Formula I through a reduction of the intermediate T-2,
in the scheme 2, R₁, R₂, R₃, R₄, A, and n are those as defined in the Formula I, except that neither R₁ nor R₂ is H; R_{b}CHO is an aldehyde compound corresponding to R₂ to be introduced in the target compound represented by Formula I.

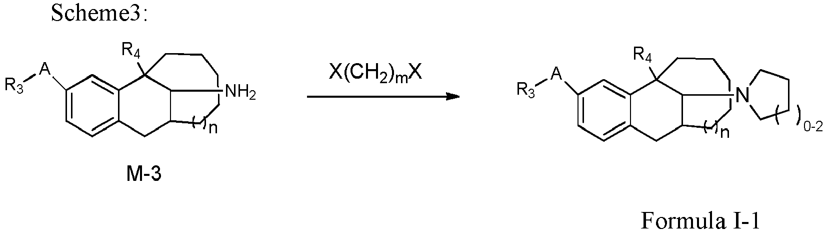

The scheme 3 including: compound M-3 reacting with compound X(CH₂)ₘX to obtain a compound represented by Formula I-1,
in the scheme 3, R₃, R₄, A, and n are those as defined in the Formula I, m is from 3 to 5; and X is F, Cl, Br, or I. 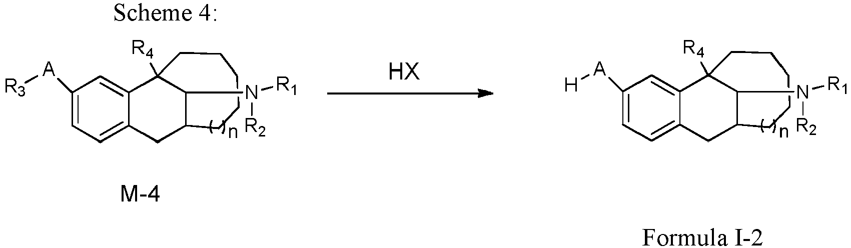
the scheme 4 including: compound M-4 reacting with HX to obtain a compound represented by Formula 1-2,
in the scheme 4, R₁, R₂, R₃, R₄, A, and n are those as defined in the Formula I, except that R₃ is not H; and X is F, Cl, Br, or I. 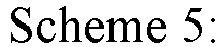 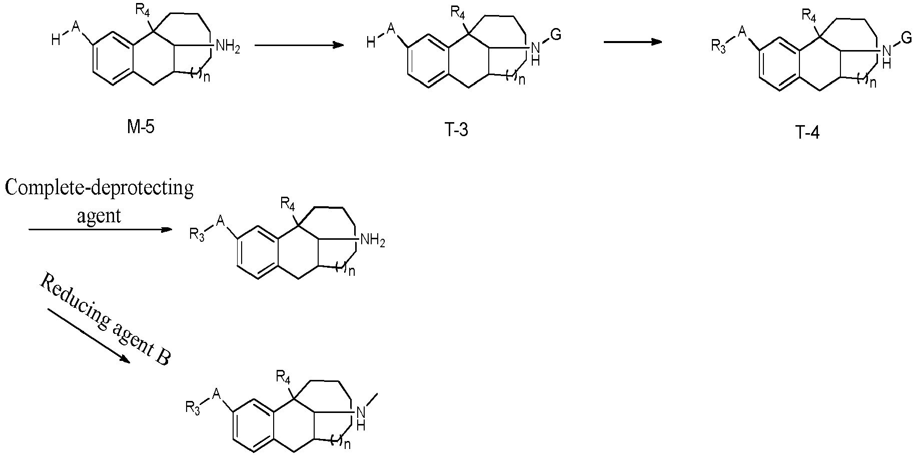
the scheme 5 including:
(1) compound M-5 reacting with an amino-protecting agent to obtain an amino-protected intermediate T-3;
(2) T-3 reacting with R₃X to obtain an intermediate T-4; and
(3) obtaining the compound represented by Formula I by completely deprotecting T-4, or obtaining a N-methylated product of the compound represented by Formula I from T-4 in presence of a reducing agent B,
in the scheme 5, R₃, R₄, A, and n are those as defined in the Formula I, except that R₃ is not H; X is F, Cl, Br, or I; and G is an amino-protecting agent.

Furthermore, those skilled in the art can understand that when G is an acyl-based protecting group (for example, t-Boc), the N-methylated product can be obtained in the presence of the reducing reagent B.

Based on the above schemes 1 to 5, in some embodiments, the following features are included.

During the reaction of the compound M-1 with the aldehyde RₐCHO or during the reaction of the compound M-2 with the aldehyde R_{b}CHO, an alcohol reagent and an organic acid are added. The alcohol reagent may be one or more selected from methanol, ethanol, propanol, or ethylene glycol. The organic acid is one or more selected from acetic acid, formic acid, or propionic acid. A molar ratio of either M-1 or M-2 to the aldehyde ranges from 1:1 to 1:15, preferably from 1:1 to 1: 11. It can be understood by those skilled in the art that, the monosubstituted or disubstituted product can be obtained by adjusting the molar ratio of M-1 to the aldehyde (for example, when the molar ratio of M-1 to the aldehyde ranges from 1:9 to 1:11, the disubstituted product can be obtained).

In the subsequent reduction reaction of the intermediate T-1 obtained from the compound M-1 or the intermediate T-2 obtained from the compound M-2, the reducing reagent A can be one or more selected from sodium cyanobohydride, sodium borohydride, or sodium borohydride acetate, preferably sodium cyanobohydride. A molar ratio of the compound M1 or the compound M2 to the reducing agent ranges from 1:1.5 to 1:8, preferably from 1:2 to 1:5.

The reduction reaction further includes post-treatment steps: adjusting the pH value of the reaction mixture to 8-10 with an alkaline reagent, adding an organic solvent (such as ethyl acetate) and separating liquid, combining organic phases, washing with a saturated salt water and drying, and obtaining the compound represented by formula I by separation through column chromatography.

The reaction of the compound M-3 with the compound X(CH₂)ₘX is preferably carried out in the presence of an organic solvent A and a basic reagent A. The organic solvent A can be one or more selected from acetonitrile, DMF, or DMSO, and preferably acetonitrile. The basic reagent can be selected from hydroxides, carbonates or bicarbonates of alkali metals or alkaline earth metals, and preferably sodium bicarbonate or potassium bicarbonate. A molar ratio of the compound M-3, X(CH₂)ₘX, and the basic reagent is 1: (2-8): (3-10), and preferably 1: (3-5): (6-8).

The reaction further includes post-treatment steps: filtrating, removing the solvent by evaporation, adding an organic solvent (such as ethyl acetate), adjusting the pH value of the reaction mixture to 8-10 with an alkaline reagent, adding an organic solvent for dilution and separating liquid, combining organic phases, washing with saturated salt water and drying, and obtaining the compound represented by Formula I by separation through column chromatography.

During the reaction of the compound M-4 with HX, 0.02 to 0.08 mmol of the compound M-4 is added to per milliliter of HX aqueous solution, preferably 0.04 to 0.06 mmol of compound M-4 is added to per milliliter of HX aqueous solution.

The reaction further includes the following post-treatment steps: removing HX by evaporation, adding an organic solvent (such as ethyl acetate), adjusting the pH value of the reaction mixture to 8-10 with an alkaline reagent, separating liquid, combining organic phases, washing with saturated salt water and drying, and obtaining the compound represented by Formula I by separation through column chromatography.

In the amino-protecting reaction of the compound M-5, G may be selected from t-butyloxycarbonyl, benzyloxycarbonyl, or p-toluenesulfonyl, and preferably t-butyloxycarbonyl. The amino-protecting reaction is carried out in the presence of an organic solvent B and a catalyst. The organic solvent B can be one or more selected from dichloromethane, carbon tetrachloride, dichloroethane, ethyl acetate, DMF, or DMSO. The catalyst can be one or more selected from DIPEA, DBU, or triethylamine, and preferably DIPEA. A molar ratio of the compound M-5 to the amino-protecting agent ranges from 1:1 to 1:2, and preferably from 1:1 to 1:1.5.

During the reaction of the obtained amino-protected intermediate T-3 with R₃X, an organic solvent C and a basic reagent C are added. The organic solvent C can be one or more selected from acetone, dioxane, methanol, ethanol, propanol, tetrahydrofuran, DMF, or DMSO, and preferably acetone. The basic reagent C can be selected from hydroxides, carbonates or bicarbonates of alkali metals or alkaline earth metals, and preferably sodium carbonate, potassium carbonate or cesium carbonate. A molar ratio of T-3 to the basic reagent ranges 0.8 to 2, preferably 1 to 1.5. The obtained intermediate T-4 is reacted in the presence of a complete-deprotecting reagent or reducing reagent B, and the deprotecting reagent is selected from TFA, formic acid, acetic acid, hydrochloric acid, sulfuric acid, or oxalic acid, and preferably TFA. The reducing reagent B adopted is selected from LiAlH₄, DIBAL-H, red Al, NaBH₄, or LiBH₄, and preferably LiAlH₄.

During the reaction, an organic solvent D is preferably added, and the organic solvent D is one or more selected from dichloromethane, trichloromethane, carbon tetrachloride, dichloroethane, tetrahydrofuran, diethyl ether, tert-butyl methyl ether, or dioxane. A feeding ratio of the intermediate T-4 to the deprotecting reagent is 1 mmol: 2-8 ml, preferably 1 mmol: 3-5 ml.

The deprotecting reaction further comprises the following post-treatment steps: removing the solvent by evaporation, adding an organic solvent (such as ethyl acetate), adjusting the pH value of the reaction mixture to 8-10 by using an alkaline reagent, separating liquid, combining organic phases, washing with saturated salt water and drying, and obtaining the compound represented by Formula I by separation through column chromatography to.

The present disclosure further provides a pharmaceutical composition, including the compound represented by Formula I, or the tautomer, optical isomer, nitrogen oxide, solvate, pharmaceutical acceptable salt or prodrug thereof as described in the present disclosure.

In some embodiments, the pharmaceutical composition of the present disclosure includes a therapeutically effective amount of the compound represented by Formula I, or the tautomer, optical isomer, nitrogen oxide, solvate, pharmaceutically acceptable salt or prodrug thereof as described in the present disclosure, and a pharmaceutically acceptable carrier.

The carrier in the pharmaceutical composition is "acceptable", which is compatible with (and preferably capable of stabilizing) the active ingredient of the composition and is unharmful to the subject being treated. One or more solubilizers can be used as pharmaceutical excipients for the delivery of active compound.

In some embodiments, the pharmaceutical composition of the present disclosure further includes a second therapeutic agent, which includes a MOR antagonist, such as naloxone, naltrexone, tramadol, samidorphan. Such a pharmaceutical composition can be used to treat opioid receptor-related disorders, such as pain, via opioid antagonist-mediated activation mechanism of MOR.

In some embodiments, the pharmaceutical composition of the present disclosure can be administered orally in any orally acceptable dosage form, including capsules, tablets, emulsions, aqueous suspensions, suppositories, sprays, inhalers, dispersions, and solutions.

In the case of tablets, commonly used carriers include lactose and corn starch. Lubricants, such as magnesium stearate, are usually added. For the capsule form, useful diluents include lactose and dried corn starch. When an aqueous suspension or emulsion is orally administered, the active ingredient can be suspended or dissolved in an oil phase combined with an emulsifier or suspending agent. If necessary, certain sweetening, flavoring or coloring agents can be added. Oral solid dosage forms can be prepared by spray drying technology; or hot melt extrusion strategy, micronization and nano-grinding technology. The spray or inhalant composition can be prepared based on the well-known technology in the field of pharmaceutical formulations. For example, the composition can be prepared as a saline solution using benzyl alcohol or other suitable preservatives, absorption enhancers to enhance bioavailability, fluorocarbons and/or other solubilizers or dispersants known in the art. The composition containing the active compound may also be applied in the form of suppositories for rectal administration.

In some embodiments, the compound of the present disclosure or the pharmaceutical composition containing the compound can be administered orally, parenterally, through the inhalation spray, topically, transrectally, nasally, buccally, transvaginally, or via implantable reservoirs. As used herein, the term "parenteral" includes subcutaneous, intradermal, intravenous, intramuscular, intraarticular, intraarterial, intrasynovial, intrasternal, intrathecal, intralesional and intracranial injection or infusion techniques. The compounds of the present disclosure and the pharmaceutical composition containing the compound have opioid receptor modulator activity and can be used to treat opioid receptor-related disorders. The disorders include pain, hyperalgesia, and cardiovascular and cerebrovascular diseases.

Therefore, the present disclosure further provides the use of the compound or the pharmaceutical composition in manufacture of a medicament for the treatment of opioid receptor-related disorders. The disorders can be pain, such as neuropathic pain or nociceptive pain. The specific types of the pain include, but are not limited to, acute pain, chronic pain, postoperative pain, neuralgia- (e.g., postherpetic neuralgia- or trigeminal neuralgia-) caused pain, diabetic neuropathy-caused pain, toothache, arthritis- or osteoarthritis-associated pain, and pain associated with cancer or treatment thereof.

Furthermore, the compound or pharmaceutical composition of the present disclosure is used to prepare analgesic drugs.

The present disclosure also provides the use of the compound represented by Formula I, or the tautomer, optical isomer, nitrogen oxide, solvate, pharmaceutically acceptable salt or prodrug thereof, or the pharmaceutical composition in manufacture of a medicament for the treatment of depression-related diseases and symptoms. The depression symptoms can be acute stress disorder, low mood adjustment disorder, Asperger's syndrome, attention deficit, bipolar disorder, borderline personality disorder, circulatory disorders, depression such as major depressive disorder (MDD) and treatment-resistant depression (TRD), dysthymic disorder, hyperactivity disorder, impulse control disorder, mixed mania, obsessive-compulsive personality disorder (OCD), paranoia, post-traumatic stress disorder, seasonal affective disorder, self-harm separation, sleep disorders, substance-induced emotional disorders, etc.

Furthermore, the compound or pharmaceutical composition as described in the present disclosure is used for manufacture of antidepressant drugs.

### Explanation of terms

The term "aliphatic hydrocarbyl" includes saturated or unsaturated, linear or branched hydrocarbon groups. The type of the aliphatic hydrocarbyl can be selected from alkyl, alkenyl, alkynyl, etc. The number of carbon atoms of the aliphatic hydrocarbyl is preferably 1-12, further preferably 1-6, including, but not limited to, the following groups: methyl, ethyl, n-propyl, isopropyl, n-butyl, isobutyl, tert-butyl, n-pentyl, isopentenyl, neopentyl, n-hexyl, vinyl, 1-propenyl, 2-propenyl, 1-methylvinyl, 1-butenyl, 1-ethylvinyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, and 1-hexynyl. The "aliphatic hydrocarbyl group" contained in other groups is the same as explained above.

The term" aryl" (or referred to as aromatic ring) refers to a monovalent group obtained after removing a hydrogen atom from an aromatic nucleus carbon of an aromatic hydrocarbon molecule, including C₆-C₁₄ aryl, and further including, but not limited to, phenyl and naphthyl.

The term "heteroaryl" refers to a heteroaromatic ring having at least one heteroatom, such as sulfur, oxygen, or nitrogen. Heteroaryl includes monocyclic systems and polycyclic systems (e.g., having 2, 3, or 4 fused rings). Examples of heteroaryl include, but are not limited to, pyridyl, pyrimidinyl, pyrazinyl, pyridazinyl, triazinyl, furyl, quinolinyl, isoquinolinyl, thienyl, imidazolyl, thiazolyl, indolyl, pyrrolyl, oxazolyl, benzofuranyl, benzothienyl, benzothiazolyl, isoxazolyl, pyrazolyl, triazolyl, tetrazolyl, indazolyl, 1,2,4-thiadiazolyl, isothiazolyl, benzothienyl, purinyl, carbazolyl, benzimidazolyl, benzoxazolyl, azabenzoxazolyl, imidazothiazolyl, benzo[1,4]dioxinyl, benzo[1,3]dioxolyl, etc. Heteroaryl preferably has 5 to 14 ring-forming atoms.

The term "cycloalkyl" should be understood to indicate a saturated monocyclic ring, bicyclic hydrocarbon ring, or bridged ring, usually having 3 to 20 carbon atoms, and preferably "C₃₋₈ cycloalkyl". The term "C₃₋₈ cycloalkyl" should be understood to indicate a saturated monocyclic or bicyclic hydrocarbon ring, having 3, 4, 5, 6, 7, or 8 carbon atoms. C₃₋₈ cycloalkyl can be a monocyclic hydrocarbon group such as cyclopropyl, cyclobutyl, cyclopentyl, cyclohexyl, cycloheptyl, cyclooctyl, or a bicyclic hydrocarbon group such as a decahydronaphthalene ring.

The term "halogen" refers to fluorine (F), chlorine (Cl), bromine (Br), or iodine (I).

The term "substituted with one or more substituents" includes, but is not limited to, substitution with one, two, three or four substituents.

The compound of the present disclosure includes a compound or a tautomer, optical isomer, nitrogen oxide, solvate, pharmaceutically acceptable salt or prodrug thereof.

The pharmaceutically acceptable salt of the compound of the present disclosure can be prepared by any suitable method provided in the literatures, and can be selected from acid addition salts, including but not limited to hydrochloride, hydrofluoride, hydrobromide, hydroiodide, sulfate, pyrosulfate, phosphate, nitrate, methanesulfonate, ethanesulfonate, 2-hydroxyethanesulfonate, benzenesulfonate, toluenesulfonate, sulfamate, 2-naphthalenesulfonate, formate, acetoacetic acid, pyruvic acid, laurate, cinnamate, benzoate, acetate, dihydroacetate, trifluoroacetate, trimethylacetate, propionate, butyrate, caproate, enanthate, undecanoate, stearate, ascorbate, camphorate, camphorsulfonate, citrate, fumarate, malate, maleate, hydroxymaleate, oxalate, salicylate, succinate, gluconate, quinate, pamoate, glycolate, tartrate, lactate, 2-(4-hydroxybenzoyl) benzoate, cyclopentane propionate, digluconate, 3-hydroxy-2-naphthoate, nicotinate, pamoate, pectinate, 3-phenylpropionate, picrate, pivalate, 4-octyl itaconate, trifluoromethanesulfonate, dodecyl sulfate, p-toluenesulfonate, naphthalene disulfonate, malonate, adipate, alginate, mandelate, glucoenanthate, glycerophosphate, sulfosalicylate, hemisulfate, thiocyanate, aspartate salt, etc.; base addition salts such as alkali metal salts, alkaline earth metal salts and ammonium salts, etc.; specifically including but not limited to: sodium salt, lithium salt, potassium salt, ammonium salt (including those formed with NH₃ and organic amine), aluminum salt, magnesium salt, calcium salt, barium salt, iron salt, ferrous salt, manganese salt, manganite salt, zinc salt, NH₄ salt, methylamine salt, trimethylamine salt, diethylamine salt, triethylamine salt, propylamine salt, tripropylamine salt, isopropylamine salt, tert-butylamine salt, N,N'-dibenzylethylenediamine salt, dicyclohexylamine salt, 1,6-hexanediamine salt, benzylamine salt, ethanolamine salt, N, N-dimethylethanolamine salt, N,N-diethylethanolamine salt, triethanolamine salt, tromethamine salt, lysine salt, arginine salt, histidine salt, glucosamine salt, N-methyl glucosamine salt, dimethyl glucosamine salt, ethyl glucosamine salt, meglumine salt, betaine salt, caffeine salt, chloroprocaine salt, procaine salt, lidocaine salt, pyridine salt, picoline salt, piperidine salt, morpholine salt, piperazine salt, purine salt, theobromine salt, choline salt, etc.

The term "solvate" refers to a form of the compound of the present disclosure, which, in a solid or liquid state, forms a complex by coordinating with solvent molecules. The hydrate is a specific form of the solvate, in which the compound is coordinated with water. In the present disclosure, the preferred solvate is a hydrate.

The term "prodrug", also referred to as "drug precursor", represents a compound to be converted in vivo into a compound represented by the aforementioned general formula or specific compound. Such conversion is affected by a hydrolysis of the prodrug in the blood, or an enzymatic conversion of the prodrug into the parent structure in the blood or tissue. The prodrug of the present disclosure may be an ester. In the present disclosure, the esters that can be used as a prodrug include phenyl esters, aliphatic (C₁₋₂₄) esters, acyloxymethyl esters, carbonates, carbamates and amino acid esters. For example, a compound in the present disclosure contains hydroxyl/carboxyl, which can be acylated to obtain a compound in the form of prodrug. Other prodrug forms include phosphate esters, for example, the compounds of phosphate esters are obtained by phosphorylating the hydroxyl group on the parent structure.

According to the positions and properties of different substituents, the compound of the present disclosure may also contain one or more asymmetric centers. Asymmetric carbon atoms can exist in the (R) or (S) configuration. When only one asymmetric center exists, a racemic mixture is produced, and when multiple asymmetric centers are contained, a diastereomer mixture is obtained. In some cases, there may be asymmetry due to hindered rotation around a specific bond, for example, the center bond connects two substituted aromatic rings of a specific compound. In addition, the substituents may also exist in *cis-* or *trans-*isomeric forms.

The compounds of the present disclosure also include all possible stereoisomers thereof, in the form of a single stereoisomer or a mixture of the stereoisomers (for example, R-isomer or S-isomer, or E-isomer or Z-isomer) in any ratio. One single stereoisomer (for example, single enantiomer or single diastereomer) of the compound of the present disclosure can be separated by any suitable method known in the art (for example, chromatography, especially chiral chromatography).

In addition, the compound may also exist in the form of tautomer. The compounds of the present disclosure include all possible tautomers of the compound of Formula (I), in the form of a single tautomer or any mixture of the tautomers in any ratio. All these isomers and mixtures thereof are included in the present disclosure.

In the present disclosure, the involved compounds also include isotopically-labeled compounds. The isotopically-labeled compounds are the same as those shown in Formula I, except that one or more are replaced by atoms having an atomic mass or mass number different from the usually naturally-occurring atomic mass or mass number. Examples of isotopes that can be incorporated into the compounds of the present disclosure include isotopes of H, C, N, O, S, F, and Cl, such as ²H, ³H, ¹³C, ¹¹C, ¹⁴C, ¹⁵N, ¹⁸O, ¹⁷O, ³²P, ³⁵S, ¹⁸F, and ³⁶Cl. The compounds of the present disclosure containing the above-mentioned isotopes and/or other isotopes of other atoms, prodrugs thereof, or pharmaceutically acceptable salts of the compounds or prodrugs are within the scope of the present disclosure. Certain isotopically-labeled compounds of the present disclosure, such as compounds incorporating radioisotopes (such as ³H and ¹⁴C), can be used for drug and/or substrate tissue distribution determination. Tritium (i.e., ³H) and carbon 14 (i.e., ¹⁴C) isotopes are particularly preferred due to their ease of preparation and detectability. Furthermore, the replacement with heavier isotopes (such as deuterium (i.e., ²H)) can provide certain therapeutic advantages derived from higher metabolic stability (for example, increased in vivo half-life or reduced dosage requirements), and therefore are preferred in certain cases. The compounds of the present disclosure as claimed in the claims can be specifically defined to be substituted with deuterium or tritium. In addition, the hydrogen existing in the substituent where deuterium or tritium is not listed separately does not mean that deuterium or tritium is excluded, but deuterium or tritium can also be included.

The term "treatment" refers to a process of applying and administering a compound to a subject for the purpose of curing, alleviating, mitigating, altering, remedying, ameliorating, or affecting a disease, disorder, or tendency. "Effective amount" refers to the amount of a compound required to impart a desired effect to a subject. As recognized by those skilled in the art, the effective amount varies depending on the route of administration, the use of excipients, and the possibility of co-use with other therapeutic treatments (such as the use of other active agents).

The beneficial effects of the present disclosure:
(1) The preferred compounds of the present disclosure have IC₅₀ values ≤ 10 µM for µ, κ and δ opioid receptors, respectively. The more preferred compounds of the present disclosure have IC₅₀ values ≤ 1 µM for µ, κ and δ opioid receptors, respectively. The further preferred compounds of the present disclosure have IC₅₀ values ≤ 100 nM for µ, κ and δ opioid receptors, respectively. The most preferred compounds of the present disclosure have IC₅₀ values ≤ 10 nM for µ, κ and δ opioid receptors, respectively.
(2) The compounds of the present disclosure have IC₅₀ values ≤ 10 µM for the µ opioid receptor. The preferred compounds of the present disclosure have IC₅₀ values ≤ 1 µM for the µ opioid receptor. The more preferred compounds of the present disclosure have IC₅₀ values ≤ 100 nM for the µ opioid receptor. The most preferred compounds of the present disclosure have IC₅₀ values ≤ 10 nM for the µ opioid receptor.
(3) The preferred compounds of the present disclosure have IC₅₀ values ≤ 10 µM for the κ opioid receptor. The more preferred compounds of the present disclosure have IC₅₀ values ≤ 1 µM for the κ opioid receptor. The further preferred compounds of the present disclosure have IC₅₀ values ≤ 100 nM for the κ opioid receptor. The most preferred compounds of the present disclosure have IC₅₀ values ≤ 10 nM for the κ opioid receptor.
(4) The preferred compounds of the present disclosure have IC₅₀ values ≤10 µM for the δ opioid receptor. The more preferred compounds of the present disclosure have IC₅₀ values ≤ 1 µM for the δ opioid receptor. The further preferred compounds of the present disclosure have IC₅₀ values ≤ 100 nM for the δ opioid receptor. The most preferred compounds of the present disclosure have IC₅₀ values ≤ 10 nM for the δ opioid receptor.
(5) The compounds of the present disclosure have selectivity for µ, κ and δ opioid receptors. For example, some compounds have selectivity for the µ opioid receptor, some compounds have selectivity for the κ opioid receptor, and some compounds have selectivity for the δ opioid receptor. More preferably, the compounds of the present disclosure have better selectivity for the µ opioid receptor. For example, the preferred compounds 4, 8, 10, 11, 13-17, 28, 29, 35, 36 and 38 of the present disclosure have selectivity for the µ opioid receptor. Among them, the selectivity of compounds 4, 8, 29, 36 and 38 is more preferred

### DESCRIPTION OF EMBODIMENTS

The preparation methods of the present disclosure will be further described in detail below in conjunction with specific examples. It should be understood that the following examples are only illustrative to explain the present disclosure, and should not be construed as limiting the scope of protection of the present disclosure. The techniques achieved by the above content of the present disclosure are all included within the scope of the present disclosure to be protected. The experimental methods used in the following examples are conventional methods unless otherwise specified. Reagents, materials and the like used in the following examples without special instructions can be obtained from commercial channels.

### Example 1: Preparation of compound 1

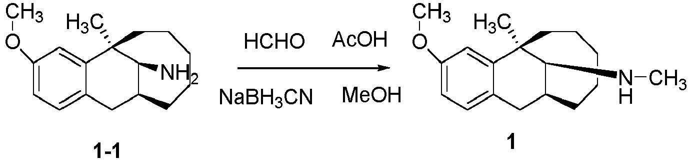

O-methyl dezocine (1-1, 0.732 mmol) was dissolved in 5 ml methanol, then a HCHO solution (0.805 mmol), NaBH₃CN (1.464 mmol) and acetic acid (0.2 ml) were sequentially added, and the mixture reacted at room temperature overnight. After the completion of massive reaction of the raw materials was monitored by TLC, ammonia water was added dropwise until pH=9. The solution was diluted with 30 ml ethyl acetate and separated, the aqueous phase was washed with ethyl acetate (30 ml), and the organic phases were combined, washed with brine, dried over anhydrous sodium sulfate, and subjected to column chromatography (DCM/MeOH=200:1) to obtain the target compound **1** (light yellow oily liquid, 120 mg, 60%).

¹H NMR (400 MHz, CDCl₃) δ 7.01 (d, *J* = 8.4 Hz, 1H), 6.77 (d, *J* = 2.5 Hz, 1H), 6.70 (dd, *J* = 8.4, 2.7 Hz, 1H), 3.80 (d, *J* = 0.6 Hz, 3H), 3.05 (dd, *J* = 16.5, 7.0 Hz, 1H), 2.69 (dd*, J=* 10.7, 5.6 Hz, 2H), 2.60 - 2.32 (m, 4H), 1.97 (t, *J* = 13.3 Hz, 1H), 1.77 - 1.34 (m, 11H), 1.10 - 0.73 (m, 3H). Ms(m/z): 274.2 [M+H].

Example 2 to Example 10: Target compounds **2** to **10** were obtained by referring to the synthesis method of compound **1**.

| **Compound No.** | **Structural formula** | **Spectrogram** |
|---|---|---|
| Compound **2** | 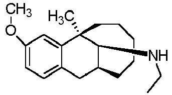 | ¹H NMR (400 MHz, CDCl₃) δ 6.99 (d, *J* = 8.4 Hz, 1H), 6.76 (d, *J* = 2.5 Hz, 1H), 6.69 (dd, *J* = 8.4, 2.6 Hz, 1H), 3.79 (s, 3H), 3.04 (dd, *J* = 16.3, 7.1 Hz, 1H), 2.92 (s, 1H), 2.79 (s, 1H), 2.67 (d, *J* = 16.5 Hz, 1H), 2.56 (s, 1H), 2.49 - 2.32 (m, 1H), 2.00 (t, *J*= 13.4 Hz, 1H), 1.76 - 1.31 (m, 10H), 1.18 - 0.69 (m, 7H). Ms(m/z): 288.2 [M+H] |
| Compound **3** | 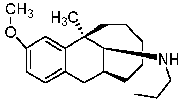 | ¹H NMR (400 MHz, CDCl₃) δ 6.99 (d, *J* = 8.4 Hz, 1H), 6.76 (d, *J* = 2.6 Hz, 1H), 6.68 (dd, *J* = 8.3, 2.6 Hz, 1H), 3.79 (s, 3H), 3.03 (dd, *J* = 16.3, 7.0 Hz, 1H), 2.89 - 2.70 (m, 2H), 2.66 (d, *J* = 16.3 Hz, 1H), 2.55 - 2.34 (m, 2H), 2.00 (t, *J* = 13.4 Hz, 1H), 1.77 - 1.43 (m, 8H), 1.38 (s, 3H), 1.14 - 0.74 (m, 7H). Ms(m/z): 302.2 [M+H] |
| Compound **4** | 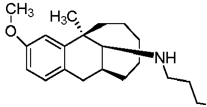 | ¹H NMR (400 MHz, CDCl₃) δ 6.99 (d, *J* = 8.4 Hz, 1H), 6.76 (d, *J* = 2.6 Hz, 1H), 6.68 (dd, *J* = 8.4, 2.6 Hz, 1H), 3.79 (s, 3H), 3.03 (dd, *J* = 16.3, 6.9 Hz, 1H), 2.93 - 2.73 (m, 2H), 2.66 (d, *J* = 16.3 Hz, 1H), 2.54 - 2.37 (m, 2H), 1.99 (t, *J* = 13.5 Hz, 1H), 1.73 - 1.34 (m, 15H), 0.90 (dt, *J* = 25.0, 10.2 Hz, 6H). Ms(m/z): 316.2 [M+H] |
| Compound **5** | 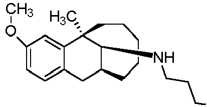 | ¹H NMR (400 MHz, CDCl₃) δ 7.01 (dd, *J* = 12.3, 7.4 Hz, 1H), 6.81 - 6.74 (m, 1H), 6.72 - 6.64 (m, 1H), 3.83 - 3.72 (m, 3H), 3.13 - 2.95 (m, 1H), 2.85 (s, 1H), 2.77 (s, 1H), 2.66 (d, *J* = 16.2 Hz, 1H), 2.46 (dd, *J* = 25.8, 5.2 Hz, 2H), 1.99 (t, *J* = 12.5 Hz, 1H), 1.65 (s, 3H), 1.55 (s, 4H), 1.38 (d, *J* = 4.9 Hz, 8H), 1.07 (s, 1H), 0.93 (d, *J* = 5.6 Hz, 6H). Ms(m/z): 330.2 [M+H] |
| Compound **6** | 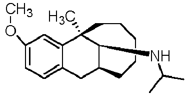 | ¹H NMR (400 MHz, CDCl₃) δ 6.99 (d, *J* = 8.4 Hz, 1H), 6.77 (d, *J* = 2.6 Hz, 1H), 6.68 (dd, *J* = 8.4, 2.7 Hz, 1H), 3.79 (s, 3H), 3.07 - 2.85 (m, 2H), 2.81 (s, 1H), 2.67 (d, *J* = 16.6 Hz, 1H), 2.39 (s, 1H), 1.95 (d, *J* = 13.1 Hz, 1H), 1.75 - 1.37 (m, 6H), 1.36 (s, 3H), 1.14 (d, *J* = 6.0 Hz, 3H), 1.07 - 0.66 (m, 8H). Ms (m/z): 302.2 [M+H] |
| Compound **7** | 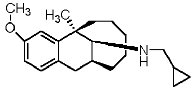 | ¹H NMR (400 MHz, CDCl₃) δ 7.10 (d, *J* = 8.3 Hz, 1H), 6.90 (s, 1H), 6.80 (d, *J* = 8.3 Hz, 1H), 3.89 (s, 3H), 3.12 (dt, *J* = 30.1, 15.1 Hz, 1H), 2.93 (d, *J* = 4.6 Hz, 1H), 2.86 - 2.57 (m, 3H), 2.51 (s, 1H), 2.16 (t, *J* = 13.3 Hz, 1H), 1.88 - 1.66 (m, 5H), 1.65 - 1.55 (m, 1H), 1.53 (s, 3H), 1.18 (ddd, *J* = 26.1, 22.0, 14.2 Hz, 3H), 1.09 - 0.91 (m, 2H), 0.61 (t, *J* = 8.4 Hz, 2H), 0.36 - 0.20 (m, 2H). Ms (m/z): 314.2 [M+H] |
| Compound **8** | 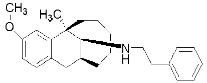 | ¹H NMR (400 MHz, CDCl₃) δ 7.29 (q, *J* = 7.9 Hz, 4H), 7.24 - 7.17 (m, 1H), 6.96 (d, *J* = 8.3 Hz, 1H), 6.74 (s, 1H), 6.66 (d, *J* = 8.2 Hz, 1H), 3.77 (d, *J* = 6.7 Hz, 3H), 3.11 (dd, *J* = 16.7, 7.0 Hz, 1H), 2.99 (dd, *J* = 16.3, 6.8 Hz, 1H), 2.87 (qd, *J* = 13.2, 6.4 Hz, 2H), 2.81 - 2.72 (m, 2H), 2.62 (d, *J* = 16.4 Hz, 1H), 2.37 (s, 1H), 1.99 - 1.86 (m, 1H), 1.64 (dd*, J* = 14.9, 6.7 Hz, 1H), 1.59 - 1.48 (m, 2H), 1.43 (dd*, J* = 13.0, 7.6 Hz, 4H), 1.32 (d, *J* = 7.1 Hz, 3H), 0.83 (d, *J* = 12.3 Hz, 3H). Ms(m/z): 364.3 [M+H] |
| Compound **9** | 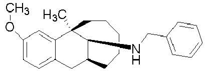 | ¹H NMR (400 MHz, CDCl₃) δ 7.52 (d, *J* = 7.5 Hz, 2H), 7.41 (t, *J* = 7.4 Hz, 2H), 7.33 (t, *J* = 7.2 Hz, 1H), 7.07 (d, *J* = 8.4 Hz, 1H), 6.84 (s, 1H), 6.76 (d, *J* = 8.3 Hz, 1H), 4.14 (d, *J* = 12.9 Hz, 1H), 3.85 (s, 3H), 3.77 (d, *J* = 12.9 Hz, 1H), 3.10 (dd, *J* = 16.4, 6.8 Hz, 1H), 2.94 (d, *J* = 4.7 Hz, 1H), 2.75 (d, *J* = 16.4 Hz, 1H), 2.59 (s, 1H), 2.10 (t, *J* = 13.4 Hz, 1H), 1.87 - 1.72 (m, 3H), 1.67 (s, 3H), 1.56 (dd*, J* = 14.0, 6.4 Hz, 1H), 1.45 (s, 3H), 1.16 (s, 1H), 1.08 - 0.86 (m, 2H). Ms(m/z): 350.3 [M+H] |
| Compound **10** | 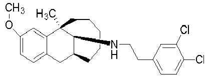 | ¹H NMR (400 MHz, CDCl₃) δ 7.44 - 7.34 (m, 2H), 7.12 (d, *J* = 8.1 Hz, 1H), 6.98 (d, *J* = 8.4 Hz, 1H), 6.75 (s, 1H), 6.68 (d, *J* = 8.3 Hz, 1H), 3.79 (s, 3H), 3.12 (s, 1H), 3.01 (dd, *J* = 16.3, 6.7 Hz, 1H), 2.89 - 2.70 (m, 4H), 2.65 (d, *J* = 16.3 Hz, 1H), 2.38 (s, 1H), 1.91 (t, *J* = 13.2 Hz, 1H), 1.73 - 1.62 (m, 1H), 1.49 (dt, *J* = 29.7, 14.1 Hz, 6H), 1.33 (s, 3H), 0.94 - 0.81 (m, 3H). Ms(m/z): 432.2 [M+H] |

### Example 11: Synthesis of compound 11

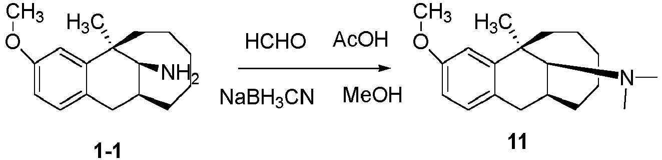

O-methyl dezocine (**1-1**, 0.732 mmol) was dissolved in 5 ml methanol, then a HCHO solution (7.32 mmol), NaBH₃CN (1.464 mmol) and acetic acid (0.2 ml) were sequentially added, and the mixture reacted at room temperature overnight. After the completion of massive reaction of the raw materials was monitored by TLC, ammonia water was added dropwise until pH=9. The solution was diluted with 30 ml ethyl acetate and separated, the aqueous phase was washed with ethyl acetate (30 ml), and the organic phases were combined, washed with brine, dried over anhydrous sodium sulfate, and subjected to column chromatography (DCM/MeOH=200:1) to obtain the target compound 11 (light yellow oily liquid, 155 mg, 74%).

¹H NMR (400 MHz, CDCl₃) δ 6.96 (d, *J =* 8.3 Hz, 1H), 6.74 (d, *J* = 2.2 Hz, 1H), 6.67 (dd*, J* = 8.3, 2.3 Hz, 1H), 3.79 (s, 3H), 3.08 (dd*, J* = 16.1, 6.4 Hz, 1H), 2.68 - 2.42 (m, 9H), 2.24 (t, *J=* 13.6 Hz, 1H), 1.95 (t, *J=* 12.7 Hz, 1H), 1.84 - 1.66 (m, 2H), 1.66 - 1.35 (m, 6H), 1.25 (dd, *J* = 21.8, 11.0 Hz, 1H), 1.08 (dd, *J* = 24.9, 12.1 Hz, 1H), 0.74 (dd, *J* = 23.6, 11.3 Hz, 1H). Ms(m/z): 288.2 [M+H].

### Example 12: Synthesis of compound 12

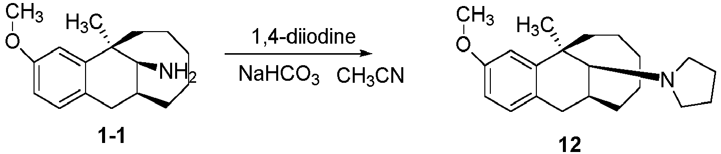

O-methyl dezocine (**1-1**, 0.732 mmol), 1,4-diiodine (2.928 mmol), and NaHCO₃ (5.124 mmol) were dissolved in 20 ml acetonitrile, refluxed overnight, and filtered after the completion of the reaction of the raw material was monitored by TLC. The solvent was removed through rotary evaporation. The remained solution was diluted with 20 ml of ethyl acetate, followed by dropwise addition of ammonia water to PH=9, dilution with 30 ml of ethyl acetate, and separation. The aqueous phase was washed with ethyl acetate (30 ml), and the organic phases were combined, washed with brine, dried over anhydrous sodium sulfate, and subjected to column chromatography (PE-PE/EA=100:1) to obtain the target compound **12** (light yellow oily liquid, 160 mg, 70%).

¹H NMR (400 MHz, CDCl₃) δ 6.95 (d, *J* = 8.4 Hz, 1H), 6.80 (s, 1H), 6.67 (d, *J*= 8.2 Hz, 1H), 3.80 (s, 3H), 3.21 (s, 1H), 2.98 (d, *J* = 16.8 Hz, 1H), 2.72 (d, *J* = 27.3 Hz, 3H), 2.58 - 2.36 (m, 3H), 2.27 (t, *J* = 12.7 Hz, 1H), 1.95 (s, 1H), 1.78 (d, *J* = 28.0 Hz, 4H), 1.68-1.37 (m, 8H), 1.26 (s, 1H), 1.23 - 1.03 (m, 1H), 0.89 (s, 1H). Ms(m/z): 314.2 [M+H].

### Example 13: Synthesis of compound 13

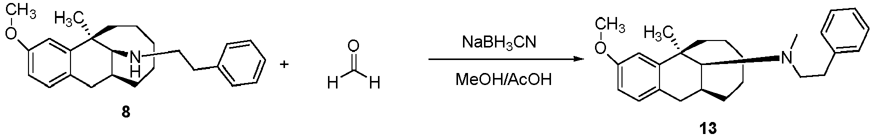

Compound **8** (0.2 mmol) was dissolved in 5 ml methanol, then a HCHO solution (1 mmol), NaBH₃CN (0.4 mmol) and acetic acid (0.2 ml) were added successively, and the mixture reacted at room temperature overnight. After the completion of massive reaction of the raw materials was monitored by TLC, ammonia water was added dropwise until pH=9. The solution was diluted with 30 ml of ethyl acetate and separated, the aqueous phase was washed with ethyl acetate (30 ml), and the organic phases were combined, washed with brine, dried over anhydrous sodium sulfate, and subjected to column chromatography (DCM/MeOH=200:1) to obtain the target compound **13** (light yellow oily liquid, 68 mg, 90%) .

¹H NMR (400 MHz, CDCl₃) δ 7.18 (t, *J* = 7.4 Hz, 2H), 7.08 (dd, *J* = 12.6, 6.9 Hz, 3H), 6.85 (d, *J* = 8.3 Hz, 1H), 6.63 (s, 1H), 6.57 (d, *J* = 8.3 Hz, 1H), 3.66 (s, 3H), 3.08 (ddd, *J* = 22.9, 17.1, 7.2 Hz, 2H), 2.84 - 2.67 (m, 4H), 2.60 (s, 3H), 2.53 - 2.39 (m, 2H), 2.06 - 1.86 (m, 2H), 1.83 - 1.71 (m, 1H), 1.56 (d, *J* = 15.8 Hz, 1H), 1.51 - 1.41 (m, 2H), 1.37 (dd*, J* = 14.7, 8.0 Hz, 1H), 1.29 (s, 3H), 1.06 (q, *J* = 12.4 Hz, 1H), 0.93 (t, *J* = 12.8 Hz, 1H), 0.65 (q, *J* = 11.7 Hz, 1H). Ms(m/z): 378.3 [M+H].

Example 14-17: Target compounds **14** to **17** were obtained by referring to the synthetic method of compound **13**.

| **Compound No.** | **Structural formula** | **Spectrogram** |
|---|---|---|
| Compound **14** | 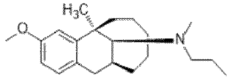 | ¹H NMR (400 MHz, CDCl₃) δ 6.97 (d, *J* = 8.5 Hz, 1H), 6.71 (dd, *J* = 19.6, 4.7 Hz, 2H), 3.79 (s, 3H), 3.14 (s, 1H), 2.86 (s, 2H), 2.56 (d, *J* = 22.2 Hz, 5H), 2.17 (s, 1H), 2.03 (d, *J* = 13.5 Hz, 1H), 1.90 (s, 1H), 1.57 (s, 6H), 1.44 (d, *J* = 10.7 Hz, 4H), 1.12 (d, *J* = 61.2 Hz, 2H), 0.92 (s, 3H), 0.74 (d, *J* = 12.3 Hz, 1H). Ms(m/z): 316.3 [M+H] |
| Compound **15** | 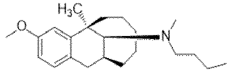 | ¹H NMR (400 MHz, CDCl₃) δ 6.95 (d, *J* = 8.3 Hz, 1H), 6.72 (d, *J* = 2.6 Hz, 1H), 6.69 - 6.63 (m, 1H), 3.78 (s, 3H), 3.13 (dd, *J* = 16.2, 7.0 Hz, 1H), 2.88 (d, *J* = 12.3 Hz, 2H), 2.57 (t, *J* = 18.6 Hz, 6H), 2.20 - 1.94 (m, 2H), 1.95 - 1.82 (m, 1H), 1.76 - 1.28 (m, 13H), 1.19 - 1.07 (m, 1H), 0.93 (t, *J* = 7.3 Hz, 3H), 0.73 (dd, *J* = 25.5, 11.4 Hz, 1H). Ms(m/z): 316.3 [M+H] |
| Compound **16** | 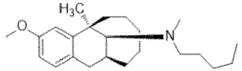 | ¹H NMR (400 MHz, CDCl₃) δ 6.97 (d, *J* = 8.4 Hz, 1H), 6.74 (d, *J* = 2.5 Hz, 1H), 6.68 (dd, *J* = 8.3, 2.5 Hz, 1H), 3.79 (s, 3H), 3.14 (dd, *J* = 16.2, 6.9 Hz, 1H), 2.97 -2.80 (m, 2H), 2.65 - 2.48 (m, 6H), 2.10 (dt, *J* = 39.7, 13.7 Hz, 2H), 1.95 - 1.82 (m, 1H), 1.69 (d, *J* = 15.8 Hz, 1H), 1.63 - 1.52 (m, 3H), 1.48 (dd, *J* = 14.5, 7.4 Hz, 2H), 1.43 - 1.37 (m, 3H), 1.37 - 1.26 (m, 4H), 1.19 (dd, *J* = 23.7, 12.7 Hz, 1H), 1.02 (dd, *J* = 25.1, 12.8 Hz, 1H), 0.92 (t, *J* = 7.0 Hz, 3H), 0.75 (dd, *J* = 25.4, 11.6 Hz, 1H). Ms(m/z): 344.3 [M+H] |
| Compound **17** | 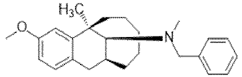 | ¹H NMR (400 MHz, CDCl₃) δ 7.53 (d, *J* = 7.3 Hz, 2H), 7.41 (t, *J* = 7.4 Hz, 2H), 7.32 (t, *J* = 7.2 Hz, 1H), 7.06 (d, *J* = 8.3 Hz, 1H), 6.84 (d, *J* = 2.0 Hz, 1H), 6.76 (dd, *J* = 8.3, 2.2 Hz, 1H), 4.35 (d, *J* = 13.6 Hz, 1H), 3.86 (d, *J* = 5.5 Hz, 3H), 3.82 (d, *J* = 13.7 Hz, 1H), 3.25 (dd, *J* = 16.1, 6.9 Hz, 1H), 3.10 (d, *J* = 3.5 Hz, 1H), 2.71 (d, *J* = 15.9 Hz, 2H), 2.54 (s, 3H), 2.42 - 2.26 (m, 1H), 2.18 (t, *J* = 13.5 Hz, 1H), 2.09 - 1.94 (m, 1H), 1.84 (dd, *J* = 15.5, 3.7 Hz, 1H), 1.66 (d, *J* = 6.9 Hz, 2H), 1.62 (s, 1H), 1.60 (d, *J* = 8.0 Hz, 3H), 1.36 (d*, J* = 9.6 Hz, 1H), 1.18 (dq, *J* = 38.9, 13.0 Hz, 2H). Ms(m/z): 364.3 [M+H] |

### Example 18: Synthesis of compound 18

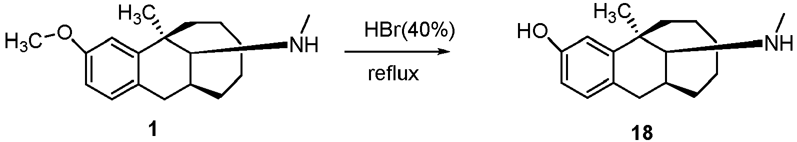

Compound **1** (0.09 mmol) was dissolved in a 40% HBr aqueous solution (2 ml), heated to react under reflux conditions for 8 hours until the complete conversion of the raw material. Most of the HBr was removed through rotary evaporation. The remained solution was diluted with 10 ml of ethyl acetate, adjusted to PH=9 with ammonia water in an ice bath, and separated. The aqueous phase was washed with EA (10 ml), and the organic phases were combined, washed with brine, dried over anhydrous sodium sulfate, and subjected to column chromatography (DCM/MeOH=200:1) to obtain the target compound **18** (light yellow oily liquid, 20 mg, 85%).

¹H NMR (400 MHz, CDCl₃) δ 6.93 (d, *J=* 8.0 Hz, 1H), 6.75 - 6.61 (m, 2H), 5.22 (s, 1H), 3.04 (dd, *J =* 16.7, 6.7 Hz, 1H), 2.88 (s, 1H), 2.69 (d, *J* = 22.1 Hz, 4H), 2.52 (s, 1H), 2.12 - 1.90 (m, 2H), 1.74 (s, 3H), 1.63 - 1.37 (m, 7H), 1.15 (d, *J=* 10.1 Hz, 1H), 0.87 (s, 2H). Ms(m/z): 260.2 [M+H].

Example 19-34: Target compounds **19** to **34** were obtained by referring to the synthetic method of compound **18**.

| **Compound No.** | **Structural formula** | **Spectrogram** |
|---|---|---|
| Compound **19** | 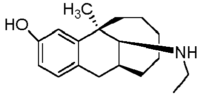 | ¹H NMR (400 MHz, CDCl₃) δ 6.93 (d, *J* = 8.2 Hz, 1H), 6.70 (s, 1H), 6.61 (d, *J* = 7.9 Hz, 1H), 3.23 (s, 1H), 3.03 (dd, *J* = 16.4, 6.6 Hz, 2H), 2.83 (s, 1H), 2.63 (dd, *J* = 25.0, 13.6 Hz, 2H), 2.43 (s, 1H), 2.12 - 1.87 (m, 1H), 1.75 - 1.35 (m, 9H), 1.16 (dd, *J*= 21.7, 14.9 Hz, 5H), 0.86 (s, 2H). Ms(m/z): 274.2 [M+H] |
| Compound **20** | 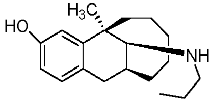 | ¹H NMR (400 MHz, CDCl₃) δ 6.92 (d, *J* = 8.2 Hz, 1H), 6.70 (s, 1H), 6.62 (d, *J* = 8.2 Hz, 1H), 3.76 (s, 1H), 3.02 (dd, *J* = 16.4, 6.9 Hz, 1H), 2.88 - 2.73 (m, 2H), 2.65 (d, *J* = 16.5 Hz, 1H), 2.58 - 2.39 (m, 2H), 2.07 - 1.92 (m, 1H), 1.75 - 1.45 (m, 8H), 1.37 (s, 3H), 1.27 (td, *J* = 7.0, 3.2 Hz, 1H), 1.15 - 0.73 (m, 6H). Ms(m/z): 288.2 [M+H] |
| Compound **21** | 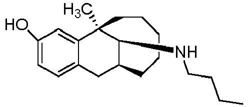 | ¹H NMR (400 MHz, CDCl₃) δ 6.93 (d,*J* = 8.2 Hz, 1H), 6.70 (s, 1H), 6.62 (d, *J* = 8.1 Hz, 1H), 3.42 (s, 1H), 3.02 (dd, *J* = 16.5, 6.8 Hz, 1H), 2.90 - 2.74 (m, 2H), 2.65 (d, *J* = 16.5 Hz, 1H), 2.57 - 2.38 (m, 2H), 2.08 - 1.89 (m, 1H), 1.73 - 1.18 (m, 15H), 1.10 - 0.78 (m, 6H). Ms(m/z): 302.2 [M+H] |
| Compound **22** | 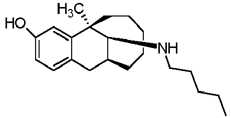 | ¹H NMR (400 MHz, CDCl₃) δ 6.93 (d, *J* = 8.1 Hz, 1H), 6.70 (s, 1H), 6.61 (d, *J* = 8.0 Hz, 1H), 4.13 (q, *J* = 7.0 Hz, 1H), 3.26 (s, 1H), 3.02 (dd, *J* = 16.3, 6.8 Hz, 1H), 2.95 - 2.81 (m, 1H), 2.78 (d, *J* = 4.1 Hz, 1H), 2.65 (d, *J* = 16.4 Hz, 1H), 2.52 (t, *J* = 12.3 Hz, 1H), 2.43 (s, 1H), 2.05 (s, 1H), 1.98 (t, *J* = 13.4 Hz, 1H), 1.65 (s, 3H), 1.55 (s, 4H), 1.36 (s, 7H), 1.26 (d, *J* = 3.1 Hz, 1H), 1.05 (d, *J* = 8.5 Hz, 1H), 0.90 (s, 4H). Ms(m/z): 316.2 [M+H] |
| Compound **23** | 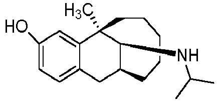 | ¹H NMR (400 MHz, CDCl₃) δ 6.92 (d, *J* = 8.2 Hz, 1H), 6.71 (s, 1H), 6.62 (d, *J* = 8.0 Hz, 1H), 3.44 - 2.92 (m, 4H), 2.84 (d, *J* = 4.5 Hz, 1H), 2.65 (d, *J* = 16.4 Hz, 1H), 2.39 (s, 1H), 1.95 (t, *J* = 13.4 Hz, 1H), 1.74 - 1.42 (m, 6H), 1.35 (s, 3H), 1.27 (d, *J* = 10.4 Hz, 1H), 1.15 (d, *J* = 6.1 Hz, 3H), 1.03 - 0.74 (m, 6H). Ms(m/z): 288.2 [M+H] |
| Compound **24** | 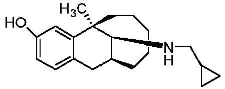 | ¹H NMR (400 MHz, DMSO) δ 9.13 (s, 1H), 9.01 (s, 1H), 6.87 (d, *J* = 8.0 Hz, 1H), 6.56 (d, *J* = 10.3 Hz, 2H), 4.43 (s, 1H), 4.07 (d, *J* = 7.9 Hz, 2H), 4.03 - 3.94 (m, 1H), 3.81 - 3.71 (m, 1H), 3.09 - 2.90 (m, 2H), 2.56 (d, *J* = 16.9 Hz, 1H), 2.38 (s, 1H), 1.89 - 1.77 (m, 1H), 1.72 (d, *J* = 16.1 Hz, 2H), 1.43 (d, *J* = 21.9 Hz, 2H), 1.38 (s, 3H), 1.31 (d, *J* = 15.8 Hz, 3H), 1.10 (d, *J* = 6.5 Hz, 2H), 0.67 (dd, *J* = 38.9, 11.4 Hz, 2H). Ms(m/z): 300.2 [M+H] |
| Compound **25** | 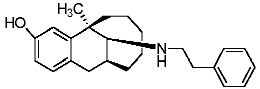 | ¹H NMR (400 MHz, DMSO) δ 8.90 (s, 1H), 7.23 (d, *J* = 6.2 Hz, 4H), 7.14 (s, 1H), 6.77 (d, *J* = 8.1 Hz, 1H), 6.52 (s, 1H), 6.44 (d, *J* = 7.3 Hz, 1H), 2.97 (s, 1H), 2.79 (dd, *J* = 31.7, 16.4 Hz, 3H), 2.64 (s, 2H), 2.45 (s, 1H), 2.25 (s, 1H), 1.81 (t, *J* = 12.7 Hz, 1H), 1.52 - 1.26 (m, 8H), 1.17 (s, 3H), 0.69 (s, 2H). Ms(m/z): 350.2 [M+H] |
| Compound **26** | 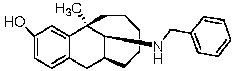 | ¹H NMR (400 MHz, CDCl₃) δ 7.35 (d, *J* = 7.3 Hz, 2H), 7.25 (t, *J* = 7.3 Hz, 2H), 7.18 (d, *J* = 7.3 Hz, 1H), 6.83 (d, *J* = 8.1 Hz, 1H), 6.61 (s, 1H), 6.52 (d, *J* = 8.1 Hz, 1H), 4.05 (q, *J* = 7.1 Hz, 1H), 3.99 (d, *J* = 12.9 Hz, 1H), 3.61 (d, *J* = 12.9 Hz, 1H), 3.27 (s, 1H), 2.92 (dd, *J* = 16.4, 6.6 Hz, 1H), 2.77 (d, *J* = 4.3 Hz, 1H), 2.57 (d, *J* = 16.4 Hz, 1H), 2.42 (s, 1H), 1.98 (s, 2H), 1.91 (t, *J* = 13.5 Hz, 1H), 1.58 (dt, *J* = 29.0, 21.5 Hz, 5H), 1.43 - 1.31 (m, 1H), 1.24 (s, 3H), 0.98 (s, 1H). Ms(m/z): 336.2 [M+H] |
| Compound **27** | 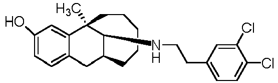 | ¹H NMR (400 MHz, DMSO) δ 8.90 (s, 1H), 7.54 (s, 1H), 7.47 (d, *J* = 8.1 Hz, 1H), 7.22 (d, *J* = 7.8 Hz, 1H), 6.76 (d, *J* = 7.8 Hz, 1H), 6.51 (s, 1H), 6.43 (d, *J* = 8.0 Hz, 1H), 2.95 (s, 1H), 2.81 (s, 1H), 2.74 (s, 2H), 2.62 (s, 2H), 2.46 (s, 1H), 2.23 (s, 1H), 1.80 (t*, J* = 12.6 Hz, 1H), 1.43 (s, 4H), 1.29 (d, *J* = 22.5 Hz, 4H), 1.23 (s, 1H), 1.16 (s, 3H), 0.69 (s, 1H). Ms(m/z): 418.2 [M+H] |
| Compound **28** | 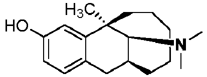 | ¹H NMR (400 MHz, CDCl₃) δ 6.91 (d, *J* = 8.2 Hz, 1H), 6.74 - 6.49 (m, 2H), 5.16 (s, 1H), 3.09 (dd, *J* = 16.1, 6.6 Hz, 1H), 2.72 - 2.51 (m, 9H), 2.29 - 2.16 (m, 1H), 1.96 (t, *J* = 13.4 Hz, 1H), 1.88 - 1.69 (m, 2H), 1.64 - 1.40 (m, 6H), 1.32 - 1.01 (m, 2H), 0.77 (dd, *J* = 23.3, 11.2 Hz, 1H). Ms(m/z): 274.2 [M+H] |
| Compound **29** | 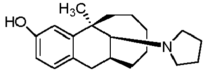 | ¹H NMR (400 MHz, CDCl₃) δ 6.88 (d, *J* = 8.1 Hz, 1H), 6.73 (s, 1H), 6.58 (dd*, J* = 8.1, 1.9 Hz, 1H), 2.95 (dd, *J* = 15.7, 4.8 Hz, 1H), 2.70 (d, *J* = 26.2 Hz, 3H), 2.61 - 2.35 (m, 3H), 2.25 (t, *J* = 13.0 Hz, 1H), 1.86 - 1.34 (m, 13H), 1.29 - 1.22 (m, 1H), 1.11 (dd, *J* = 25.7, 12.2 Hz, 1H), 0.89 (d, *J* = 5.7 Hz, 1H). Ms(m/z): 300.2 [M+H] |
| Compound **30** | 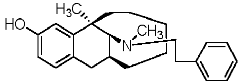 | ¹H NMR (400 MHz, CDCl₃) δ 7.20 (t, *J* = 7.2 Hz, 2H), 7.15 - 7.07 (m, 3H), 6.81 (d, *J* = 8.2 Hz, 1H), 6.58 (s, 1H), 6.52 (d, *J* = 8.1 Hz, 1H), 5.35-3.29 (m, 1H), 3.08 (ddd, *J* = 22.5, 16.2, 7.0 Hz, 2H), 2.88 - 2.67 (m, 4H), 2.62 (s, 3H), 2.54 - 2.39 (m, 2H), 1.97 (dt, *J* = 28.1, 13.6 Hz, 2H), 1.84 - 1.72 (m, 1H), 1.54 (d, *J* = 16.2 Hz, 1H), 1.51 - 1.43 (m, 2H), 1.41 - 1.32 (m, 1H), 1.28 (s, 3H), 1.07 (dd, *J* = 23.9, 12.6 Hz, 1H), 0.93 (q, *J* = 12.5 Hz, 1H), 0.75 - 0.60 (m, 1H). Ms(m/z): 364.2 [M+H] |
| Compound **31** | 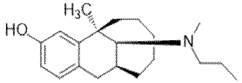 | ¹H NMR (400 MHz, CDCl₃) δ 6.90 (d, *J* = 8.2 Hz, 1H), 6.71 (s, 1H), 6.63 (s, 1H), 3.12 (s, 1H), 2.89 (s, 2H), 2.56 (d, *J* = 27.4 Hz, 4H), 2.19 (d, *J* = 19.1 Hz, 1H), 2.05 - 1.84 (m, 3H), 1.64 (d, *J* = 27.6 Hz, 11H), 1.25 (s, 2H), 0.92 (s, 3H), 0.82 - 0.65 (m, 1H). Ms(m/z): 302.3 [M+H] |
| Compound **32** | 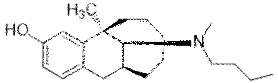 | ¹H NMR (400 MHz, CDCl₃) δ 6.90 (d, *J* = 8.3 Hz, 1H), 6.67 (s, 1H), 6.59 (d, *J* = 8.2 Hz, 1H), 3.12 (dd, *J* = 16.0, 68 Hz, 1H), 2.87 (d, *J* = 13.2 Hz, 2H), 2.68 - 2.51 (m, 6H), 2.20 - 1.96 (m, 3H), 1.99 - 1.81 (m, 2H), 1.58 (dd, *J* = 25.3, 14.3 Hz, 4H), 1.50 -1.27 (m, 8H), 1.20 -1.13 (m, 1H), 1.03 (s, 1H), 0.93 (dd, *J* = 8.1, 6.4 Hz, 3H), 0.82 - 0.66 (m, 1H). Ms(m/z): 316.3 [M+H] |
| Compound **33** | 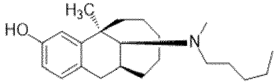 | ¹H NMR (400 MHz, CDCl₃) δ 6.90 (d, *J* = 8.0 Hz, 1H), 6.69 (s, 1H), 6.61 (d, *J* = 80 Hz, 1H), 3.12 (dd, *J* = 15.9, 6.3 Hz, 1H), 2.87 (d, *J* = 13.4 Hz, 2H), 2.59 (s, 3H), 2.55 (d, *J* = 11.9 Hz, 3H), 2.09 (dt, *J* = 35.1, 13.4 Hz, 2H), 1.94 - 1.81 (m, 1H), 1.65 (d, *J* = 15.7 Hz, 1H), 1.56 (d, *J* = 9.4 Hz, 3H), 1.48 (dd, *J* = 13.5, 7.5 Hz, 2H), 1.38 (s, 3H), 1.36 - 1.25 (m, 5H), 1.22- 1.11 (m, 1H), 1.10 - 0.99 (m, 1H), 0.92 (t, *J* = 6.2 Hz, 3H), 0.77 (q, *J* = 12.2 Hz, 1H). Ms(m/z): 330.3 [M+H] |
| Compound **34** | 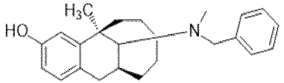 | ¹H NMR (400 MHz, CDCl₃) δ 7.48 (d, *J* = 7.4 Hz, 2H), 7.36 *(t, J=* 7.4 Hz, 2H), 7.28 (t*, J* = 7.7 Hz, 1H), 6.94 (d, *J* = 8.2 Hz, 1H), 6.72 (d, *J* = 2.5 Hz, 1H), 6.64 (dd, *J* = 8.2, 2.5 Hz, 1H), 4.30 (d, *J* = 13.6 Hz, 1H), 3.77 (d, *J* = 13.7 Hz, 1H),3.18 (dd, *J* = 16.2, 7.0 Hz, 1H), 3.04 (d, *J* = 3.7 Hz, 1H), 2.64 (d, *J* = 15.9 Hz, 2H), 2.50 (s, 3H), 2.29 - 2.17 (m, 1H), 2.11 (dd, *J* = 18.5, 8.9 Hz, 1H), 2.00 - 1.89 (m, 1H), 1.74 (d, *J* = 15.4 Hz, 1H), 1.58 (dd, *J* = 17.2, 5.7 Hz, 3H), 1.51 (s, 3H), 1.30 (d, *J* = 8.3 Hz, 2H), 1.21 - 1.02 (m, 2H). Ms(m/z): 350.2 [M+H] |

### Example 35: Synthesis of compound 35

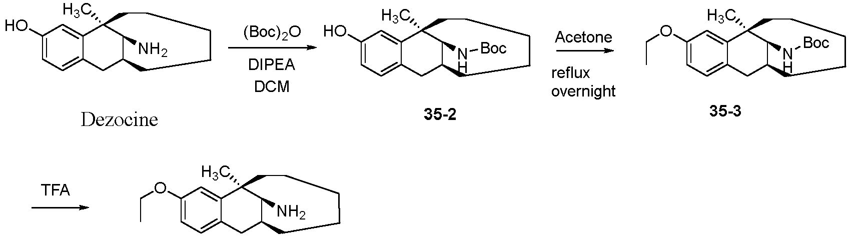

### Step 1: Synthesis of compound 35-2

**Dezocine** (4.08 mmol) was dissolved in 10 ml of dichloromethane, DIPEA (12.24 mmol) was added, (Boc)₂O (4.50 mmol) dissolved in 5 ml of dichloromethane was added dropwise under an ice bath, followed by heating to room temperature to react overnight. The reaction solution was diluted with 50 ml dichloromethane, washed with 2N HCl (20 ml X 2), washed with brine (10 ml), dried over anhydrous sodium sulfate, and subjected to column chromatography (PE/EA=100:1) to obtain compound **35-2** (light yellow oily liquid, 1.12 g, yield 80%).

### Step 2: Synthesis of compound 35-3

Compound **35-2 (**1 mmol) was dissolved in 10 ml acetone, then potassium carbonate (1.2 mmol) and halogenated alkanes (1.1 mmol) were added, followed by refluxing overnight, cooling to room temperature, drying through rotary evaporation, and subjecting to column chromatography to obtain compound **35-3** (light yellow oily liquid, 316 mg, 80%).

¹H NMR (400 MHz, CDCl₃) δ 6.99 (d, *J=* 8.2 Hz, 1H), 6.73 (dd, *J=* 28.4, 8.0 Hz, 2H), 4.95 (d, *J=* 9.9 Hz, 1H), 4.04 (ddt, *J=* 13.7, 11.2, 5.8 Hz, 3H), 3.18 (dd, *J=* 16.2, 6.6 Hz, 1H), 2.62 (d, *J=* 16.3 Hz, 1H), 2.31 (s, 1H), 1.89 - 1.21 (m, 25H), 0.95 *(d, J=* 51.2 Hz, 3H). Ms(m/z): 396.2 [M+Na]

### Step 3: Synthesis of compound 35

Compound **35-3** (0.28 mmol) was dissolved in 5 ml DCM, TFA (1 ml) was added dropwise to react for 10 minutes, followed by removing TFA through rotary evaporation, dilution with 20 ml of EA, dropwise addition of ammonia to pH=9, and separation. The aqueous was washed with EA (20 ml X 2), and the organic phases were combined, washed with brine, dried over anhydrous sodium sulfate, and subjected to column chromatography to obtain target compound **35** (light yellow oily liquid, 70 mg, 91%).

¹H NMR (400 MHz, CDCl₃) δ 6.99 (d*, J* = 8.3 Hz, 1H), 6.77 (d, *J* = 2.1 Hz, 1H), 6.74 - 6.61 (m, 1H), 4.01 (dt, *J=* 11.3, 5.7 Hz, 2H), 3.37 - 3.00 (m, 2H), 2.66 (d, *J=* 16.6 Hz, 1H), 2.31 (s, 1H), 2.11 - 1.90 (m, 1H), 1.75 (s, 3H), 1.67 - 1.31 (m, 9H), 1.08 (s, 1H), 0.91 - 0.67 (m, 2H). Ms(m/z): 274.2 [M+H].

Compound **36-3** was obtained by referring to the synthesis method of compound **35-3**.

| **Compound No.** | **Structural formula** | **Spectrogram** |
|---|---|---|
| Compound **36-3** | 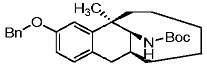 | ¹H NMR (400 MHz, CDCl₃) δ 7.44 (d, *J* = 7.2 Hz, 2H), 7.38 (t, *J* = 7.3 Hz, 2H), 7.33 (d, *J* = 7.0 Hz, 1H), 7.00 (d, *J* = 8.4 Hz, 1H), 6.84-6.73 (m, 2H), 5.04 (d, *J* = 3.0 Hz, 2H), 4.94 (d, *J* = 10.0 Hz, 1H), 4.09 (dd, *J* = 10.2, 4.9 Hz, 1H), 3.18 (dd, *J* = 16.4, 6.8 Hz, 1H), 2.62 (d, *J* = 16.4 Hz, 1H), 2.31 (s, 1H), 1.89 -1.53 (m, 5H), 1.46 (d, *J* = 20.9 Hz, 9H), 1.36 - 1.20 (m, 5H), 0.99 (s, 3H). Ms(m/z): 458.2 [M+Na] |

Example 36: Compound **36** was obtained by referring to the synthesis method of compound 35.

| **Compound No.** | **Structural formula** | **Spectrogram** |
|---|---|---|
| Compound **36** | 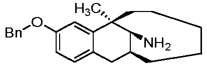 | ¹H NMR (400 MHz, CDCl₃) δ 7.55 -7.27 (m, 5H), 7.00 (d, *J* = 8.3 Hz, 1H), 6.88 - 6.72 (m, 2H), 5.04 (s, 2H), 3.21 - 2.91 (m, 2H), 2.65 (d, *J* = 16.6 Hz, 1H), 2.22 (s, 1H), 1.99 (dd, *J* = 28.0, 14.3 Hz, 1H), 1.80- 1.32 (m, 11H), 1.09 - 0.57 (m, 3H). Ms(m/z): 336.2 [M+H] |

### Example 37: Synthesis of compound 37

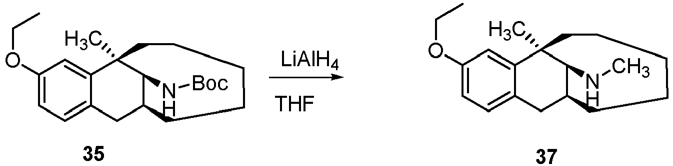

Compound **35** (0.28 mmol) was dissolved in 5 ml THF, a solution of LiAlH₄ in tetrahydrofuran (1M, 4eq) was added dropwise under an ice bath, followed by heating to 60°C to react overnight. Then, the reaction solution was cooled in an ice bath, water (10 ml) was added dropwise, IN NaOH solution (10 ml) was added, diluted with 30 ml of EA, and separation. The aqueous phase was washed with EA (30ml), the organic phases were combined, washed with brine, dried over anhydrous sodium sulfate, and subjected to column chromatography to obtain target compound **37** (light yellow oily liquid, 56 mg, 69%).

¹H NMR (400 MHz, CDCl₃) δ 6.86 *(d, J=* 8.3 Hz, 1H), 6.63 (s, 1H), 6.55 (d, *J* = 8.3 Hz, 1H), 4.04 - 3.74 (m, 2H), 2.91 (dd, *J=* 16.5, 6.9 Hz, 1H), 2.64 - 2.48 (m, 2H), 2.45 - 2.32 (m, 4H), 1.82 (t, *J* = 13.4 Hz, 1H), 1.66 - 1.18 (m, 13H), 1.02 - 0.54 (m, 3H). Ms(m/z): 288.2 [M+H].

Example 38: Compound **38** was obtained by referring to the synthesis method of compound **37.**

| **Compound No.** | **Structural formula** | **Spectrogram** |
|---|---|---|
| Compound **38** | 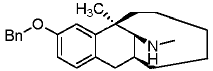 | ¹H NMR (400 MHz, CDCl₃) δ 7.49 -7.20 (m, 6H), 6.81 - 6.57 (m, 2H), 5.14 - 4.85 (m, 2H), 3.00 (dd, *J* = 16.5, 7.0 Hz, 1H), 2.74 - 2.40 (m, 7H), 1.91 (t, *J* = 13.4 Hz, 2H), 1.71 - 1.12 (m, 15H), 1.17 - 0.63 (m, 3H). Ms(m/z): 350.2 [M+H] |

### Example 39: Synthesis of compound 39

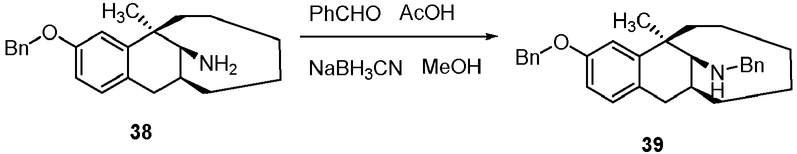

Compound **38** (0.3 mmol) was dissolved in 5 ml methanol, then benzaldehyde (3 mmol), NaBH₃CN (1.5 mmol) and acetic acid (0.2 ml) were added sequentially, and the mixture reacted at room temperature overnight. After the completion of massive reaction of the raw materials was monitored by TLC, ammonia water was added dropwise until pH=9. The solution was diluted with 30 ml of ethyl acetate and separated, the aqueous phase was washed with ethyl acetate (30 ml), and the organic phases were combined, washed with brine, dried over anhydrous sodium sulfate, and subjected to column chromatography (PE/EA=200:1) to obtain the target compound **39** (light yellow oily liquid, 89 mg, 70%). ¹H NMR (400 MHz, CDCl₃) δ 7.35 (dd, *J=* 37.1, 31.4 Hz, 10H), 6.98 (s, 1H), 6.82 (s, 1H), 6.75 (s, 1H), 5.02 (s, 2H), 4.08 (d, *J=* 11.7 Hz, 1H), 3.69 (d, *J=* 12.5 Hz, 1H), 3.01 (d, *J=* 15.9 Hz, 1H), 2.86 (s, 1H), 2.67 (d, *J* = 16.9 Hz, 1H), 2.50 (s, 1H), 2.00 (s, 1H), 1.62 *(d, J=* 43.5 Hz, 6H), 1.38 *(d, J=* 33.2 Hz, 5H), 1.07 (s, 1H), 0.86 (d, *J=* 33.1 Hz, 2H). Ms(m/z): 426.3 [M+H].

### Example 40: Synthesis of compound 40

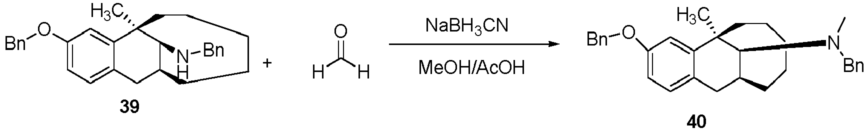

Compound **39** (0.15 mmol) was dissolved in 5 ml methanol, then a HCHO solution (1 mmol), NaBH₃CN (0.5 mmol) and acetic acid (0.2 ml) were added sequentially, and the mixture reacted at room temperature overnight. After the completion of massive reaction of the raw materials was monitored by TLC, ammonia water was added dropwise until pH=9. The solution was diluted with 30 ml ethyl acetate and separated, the aqueous phase was washed with ethyl acetate (30 ml), and the organic phases were combined, washed with brine, dried over anhydrous sodium sulfate, and subjected to column chromatography (PE/EA=200:1) to obtain the target compound **40** (light yellow oily liquid, 40 mg, 60%).

¹H NMR (400 MHz, CDCl₃) δ 7.57 -7.26 (m, 10H), 6.98 (d, *J=* 8.2 Hz, 1H), 6.86 - 6.62 (m, 2H), 5.14-4.91 (m, 2H), 4.27 (s, 1H), 3.77 (s, 1H), 3.09 (d, *J=* 52.7 Hz, 2H), 2.64 (d, *J=* 17.1 Hz, 2H), 2.47 (s, 2H), 2.23 (t, *J* = 13.7 Hz, 1H), 2.07 (d, *J=* 13.1 Hz, 1H), 1.97 (s, 1H), 1.73 (s, 1H), 1.51 *(d, J=* 14.9 Hz, 6H), 1.26 (s, 1H), 1.13 (s, 1H), 1.05 - 0.72 (m, 2H). Ms(m/z): 440.3 [M+H]

### Example 41: In vitro activity assay

### 1. Purpose

Through the radioisotope ligand competitive binding assay, IC50 of the compound was used as an indicator to evaluate the affinity of the compound to µ, κ and δ opioid receptors.

2. Experimental Materials

(1) Reagents

The cell membrane was extracted from stably transfected cells constructed by WuXi AppTec, Shanghai.
3H-diprenophrine (PerkinElmer, Cat: NET1121250UC, Lot: 2143599)
3H-DAMGO (PerkinElmer, Cat: NET902250UC, Lot: 2139100)
3H-DADLE (PerkinElmer, Cat: NET648250UC, Lot: 2060549)
Tris base (Sigma, Cat: T6066-1KG), prepare 1M stock and adjust pH to 7.4.
0.5M EDTA (Invitrogen, Cat: 15575-038)
1M MgCl2 (Sigma, Cat: M1028-100ml)
PEI (Poly ethyleneimine) (Sigma, Cat: P3143)
Microscint 20 cocktail (PerkinElmer, Cat: 6013329)
Naltrindole (Sigma, Cat; N115)
(±)trans-U-50488 (Sigma, Cat: D8040)
DAMGO (Sigma, Cat: E7384)

### (2) Experiment buffer and wash buffer

| Target | Experiment buffer | Plate washing buffer |
|---|---|---|
| Op-delta | 50 mM Tris-HCl pH7.4, 10 mM MgCl2, 1 mM EDTA | 50 mM Tris-HCl pH 7.4, stored at 4°C |
| Op-kappa | 50 mM Tris-HCl pH 7.4 | |
| Op-mu | 50 mM Tris-HCl pH 7.4, 5 mM MgCl₂ | |

### (3) Consumables and instruments

GF/C filter plate, Perkin Elmer (Cat#6005174)
96-well plate, Agilent (Cat#5042-1385)
Plate sealing film, Perkin Elmer (Cat#6005250)
MicroBeta2 (PerkinElmer)
Cell harvest C961961, (Perkin Elmer)

### 3. Method steps

### 1) Cell membrane and radioisotope preparation

| Target | Cell membrane protein concentration (µg/well) | Radioisotope | Final radioisotope concentration (nM) |
|---|---|---|---|
| DOR | 6.7 | [3H]-DADLE | 0.5 |
| MOR | 20 | [3H]DAMGO | 0.5 |
| KOR | 6.7 | [3H]Diprenorphine | 0.3 |

### 2) Compound preparation

| Target | Initial compound concentration in compound plate (mM) | Final compound concentration (nM) | Initial concentration of positive compound (mM) | Final concentration of positive compound (nM) | Concentration of non -specific binding well compound |
|---|---|---|---|---|---|
| DOR | 2 | 10000 | 0.02 | 100 | Naltrindole (1 µM) |
| MOR | 2 | 10000 | 0.2 | 1000 | Naltrindole (1 µM) |
| KOR | 2 | 10000 | 0.2 | 1000 | Trans-U-50488 (5 µM) |

### 3) Experimental steps

(1) 1 µL of the test compounds, negative control (i.e., DMSO) and positive control (i.e., non-specific binding well compound), after being prepared, were respectively transferred to a 96-well plate;
(2) 99 µL of the prepared opioid receptor membrane protein was added to each well of the 96-well plate containing 1 µL of the compound;
(3) 100 µL of 2x corresponding radioisotope ligand was added to each well;
(4) The plate was placed on a shake and incubated at room temperature for 1 hour;
(5) Each well of the GF/C plate was soaked with 50 µL of 0.3% PEI for at least half an hour in advance;
(6) After the incubation was finished, the GF/C plate was washed with plate washing buffer once by using Harvest. Then, the cell membranes in the 96-well plate were collected onto the GF/C plate using Harvest, and the GF/C plate was washed four times with the plate washing buffer, each time about 250 µL;
(7) The GF/C plate was placed in an oven at 50°C for 1 hour;
(8) The bottom of the GF/C plate was sealed with a plate sealing film, 50 µL of Microscint-20 scintillation fluid was added to each well, and then the plate was sealed with a transparent sealing film for microplate;
(9) The radioactive signal value CPM was read using MicroBeta2;
(10) The data was analyzed with Prism 5. The percentage inhibition rate was calculated with the calculation formula: %Inh = (1-Background subtracted Assay value/Background subtracted HC value)^{∗} 100.

The following table illustrates the IC₅₀ values of the tested compounds against µ, κ and δ opioid receptors.

**Table 41: IC₅₀ values of compounds for µ, κ and δ opioid receptors**

| Test samples | µ IC₅₀ | κ IC₅₀ | δ IC₅₀ |
|---|---|---|---|
| Hydrochloride of compound **1** | C | D | E |
| Hydrochloride of compound **2** | C | D | E |
| Hydrochloride of compound **3** | B | C | E |
| Hydrochloride of compound **4** | B | D | D |
| Hydrochloride of compound **5** | C | D | E |
| Hydrochloride of compound **6** | D | D | E |
| Hydrochloride of compound **7** | C | C | E |
| Hydrochloride of compound **8** | B | E | E |
| Hydrochloride of compound **9** | C | C | D |
| Hydrochloride of compound **10** | C | D | E |
| Hydrochloride of compound **11** | C | E | E |
| Hydrochloride of compound **12** | D | E | E |
| Hydrochloride of compound **13** | C | E | E |
| Hydrochloride of compound **14** | C | D | E |
| Hydrochloride of compound **15** | C | D | E |
| Hydrochloride of compound **16** | C | E | E |
| Hydrochloride of compound **17** | C | E | E |
| Hydrochloride of compound **18** | B | C | D |
| Hydrochloride of compound **19** | A | B | D |
| Hydrochloride of compound **20** | A | B | C |
| Hydrochloride of compound **21** | A | B | B |
| Hydrochloride of compound **22** | A | B | B |
| Hydrochloride of compound **23** | B | C | D |
| Hydrochloride of compound **24** | B | C | D |
| Hydrochloride of compound **25** | A | C | C |
| Hydrochloride of compound **26** | A | A | A |
| Hydrochloride of compound **27** | B | B | C |
| Hydrochloride of compound **28** | A | C | D |
| Hydrochloride of compound **29** | B | D | D |
| Hydrochloride of compound **30** | A | B | B |
| Hydrochloride of compound **31** | A | C | B |
| Hydrochloride of compound **32** | A | B | B |
| Hydrochloride of compound **33** | A | C | C |
| Hydrochloride of compound **34** | A | A | B |
| Hydrochloride of compound **35** | C | E | E |
| Hydrochloride of compound **36** | B | D | D |
| Hydrochloride of compound **37** | D | D | E |
| Hydrochloride of compound **38** | B | D | D |
| Hydrochloride of compound **39** | A | C | C |
| Hydrochloride of compound **40** | A | A | B |

| | | | |
|---|---|---|---|
| A: <10 nM; 10 nM<B<100 nM; 100 nM<C<1 µM; 1 µM <D<10 µM; E: >10 µM | | | |

The in vitro activity assays substantiate that the compounds of the present disclosure have IC50 values ≤ 10 µM for µ, κ and δ opioid receptors, respectively. The more preferred compounds of the present disclosure have IC50 values ≤ 1 µM for µ, κ and δ opioid receptors, respectively. The further preferred compounds of the present disclosure have IC50 values ≤ 100 nM for µ, κ and δ opioid receptors, respectively.

The in vitro activity assay also indicated that compounds 19-22, 25, 26, 28, 30-34, 39 and 40, and particularly, compounds 21, 22, 25, 26, 30, 33-34 and 40, are compounds of the present disclosure with higher activity. Among these compounds, the IC₅₀ values for at least one of the µ, κ, and δ opioid receptors can reach Grade A, more preferably, the IC₅₀ values for at least two of the opioid receptors can reach Grade A, and most preferably, the IC₅₀ values for all of the three opioid receptors can reach A grade.

The most preferred compounds of the present disclosure have IC50 values ≤ 10 nM for µ, κ, and δ opioid receptors, respectively; meanwhile, the compounds of the present disclosure have selectivity for µ, κ, and δ opioid receptors, and more preferably, the compounds of the present disclosure have better selectivity for the µ opioid receptor. For example, the preferred compounds 4, 8, 10, 11, 13-17, 28, 29, 35, 36 and 38 of the present disclosure have selectivity for the µ opioid receptor. Among them, the selectivity of compounds 4, 8, 29, 36 and 38 is more preferred.

### Example 42. In vivo pharmacodynamic investigation

A mode of pain induced by heat radiation in mice and a mode of pain induced by hot plate in mice were used to evaluate the analgesic intensity of the test compounds. The results in Table 42 show that compound 26 has the strongest analgesic effect in the four mouse models of pain, followed by compound 22, all of which are stronger than dezocine.

**[Table 42]**

| **Test substance** | **Light and heat-induced pain** | | | | **Hot plate-induced pain** | |
|---|---|---|---|---|---|---|
| | **ED₅₀ (mg/kg)** | | **ED₉₅ (mg/kg)** | | **ED₅₀ (mg/kg)** | **ED₉₅ (mg/kg)** |
| | **Male** | **Female** | **Male** | **Female** | **Male** | **Female** |
| Hydrochloride of compound 21 | 0.12 | 0.068 | 0.18 | 0.083 | 0.15 | - |
| Hydrochloride of compound 25 | 0.74 | 1.15 | 1.18 | 2.88 | - | - |
| Hydrochloride of compound 26 | 0.017 | 0.025 | 0.021 | 0.043 | 0.045 | - |
| Hydrochloride of compound 22 | 0.13 | 0.18 | 0.26 | 0.26 | 0.3 | - |

### Example 43. Pharmacokinetic investigation of intravenous administration in mice and rats

Male mice were injected intravenously with the compound of the present disclosure, and the concentration of the unchanged compound in plasma and the concentration of the metabolite of dezocine hydrochloride were measured after the administration.

The results prove that dezocine hydrochloride has a faster clearance rate in mouse plasma, with a T_{1/2} of 0.903h. Compounds 21, 22, 25, and 26 can be cleared slower than the dezocine hydrochloride in mouse plasma, with T_{1/2} of 1.27h, 1.24h, 1.65h, 1.35h, respectively.

The embodiments of the present disclosure are described above. However, the present disclosure is not limited to the above-mentioned embodiments. Any modification, equivalent replacement, improvement, etc. made within the spirit and principle of the present disclosure should be included in the protection scope of the present disclosure.

## Claims

1. A compound represented by Formula I, or a tautomer, optical isomer, nitrogen oxide, solvate, pharmaceutically acceptable salt or prodrug thereof, 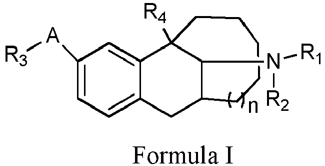
wherein R₁ and R₂ are each independently selected from H, C₁-C₁₂ aliphatic hydrocarbyl, C₆-C₁₄ aryl, C₆-C₁₄ aryl-C₁-C₁₂ aliphatic hydrocarbyl, C₃-C₈ cycloalkyl, C₃-C₈ cycloalkyl-Ci-C₁₂ aliphatic hydrocarbyl, five- to fourteen-membered heteroaryl, or five- to fourteen-membered heteroaryl-C₁-C₁₂ aliphatic hydrocarbyl, wherein the C₆-C₁₄ aryl, the C₃-C₈ cycloalkyl, and the five- to fourteen-membered heteroaryl are optionally substituted with one or more halogens, -OH groups, C₁-C₁₂ aliphatic hydrocarbyl groups, C₁-C₁₂ aliphatic hydrocarbyl oxyl groups, or C₁-C₁₂ aliphatic hydrocarbyl-S- groups; or wherein R₁, R₂ and N connected thereto together form a N-containing four- to six-membered ring, and the N-containing four- to six-membered ring is optionally substituted by one or more halogens, -OH groups, C₁-C₁₂ aliphatic hydrocarbyl groups, C₁-C₁₂ aliphatic hydrocarbyl oxyl groups, or C₁-C₁₂ aliphatic hydrocarbyl-S- groups;
R₃ is selected from H, C₁-C₁₂ aliphatic hydrocarbyl, C₆-C₁₄ aryl, C₆-C₁₄ aryl-C₁-C₁₂ aliphatic hydrocarbyl, C₃-C₈ cycloalkyl, C₃-C₈ cycloalkyl-C₁-C₁₂ aliphatic hydrocarbyl, five-to fourteen-membered heteroaryl, or five- to fourteen-membered heteroaryl-C₁-C₁₂ aliphatic hydrocarbyl, wherein the C₆-C₁₄ aryl, the C₃-C₈ cycloalkyl, and the five- to fourteen-membered heteroaryl are optionally substituted by one or more halogens, C₁-C₁₂ aliphatic hydrocarbyl groups, C₁-C₁₂ aliphatic hydrocarbyl oxyl groups, or C₁-C₁₂ aliphatic hydrocarbyl-S- groups;
R₄ is selected from H, OH, halogen, C₁-C₁₂ aliphatic hydrocarbyl, C₁-C₁₂ aliphatic hydrocarbyl oxyl, or C₁-C₁₂ aliphatic hydrocarbyl-S-;
at least one of R₁, R₂, or R₃ is not H;
A is selected from O or S; and
n is selected from 0, 1 or 2.

2. The compound represented by Formula I, or the tautomer, optical isomer, nitrogen oxide, solvate, pharmaceutically acceptable salt or prodrug thereof according to claim 1, wherein R₁ and R₂ are each independently selected from H, C₁-C₁₂ aliphatic hydrocarbyl, C₆-C₁₄ aryl-C₁-C₆ aliphatic hydrocarbyl, C₃-C₈ cycloalkyl, or C₃-C₈ cycloalkyl-C₁-C₆ aliphatic hydrocarbyl, wherein the C₆-C₁₄ aryl and the C₃-C₈ cycloalkyl are optionally substituted by one or more halogens, -OH groups, C₁-C₆ aliphatic hydrocarbyl groups, C₁-C₆ aliphatic hydrocarbyl oxyl groups, or C₁-C₆ aliphatic hydrocarbyl-S- groups; or, R₁, R₂ and N connected thereto together form a N-containing four- to six-membered ring; R₃ is selected from H, C₁-C₆ aliphatic hydrocarbyl, or C₆-C₁₄ aryl-C₁-C₁₂ aliphatic hydrocarbyl, wherein the C₆-C₁₄ aryl is optionally substituted by one or more halogens, C₁-C₆ aliphatic hydrocarbyl groups, C₁-C₆ aliphatic hydrocarbyl oxyl groups, or C₁-C₆ aliphatic hydrocarbyl-S- groups; R₄ is selected from C₁-C₆ aliphatic hydrocarbyl, C₁-C₆ aliphatic hydrocarbyl oxyl, or C₁-C₆ aliphatic hydrocarbyl-S-; at least one of R₁, R₂, or R₃ is not H; A is selected from O or S; and n is selected from 1 or 2.

3. The compound represented Formula I, or the tautomer, optical isomer, nitrogen oxide, solvate, pharmaceutically acceptable salt or prodrug thereof according to claim 1 or 2, wherein R₁ and R₂ are each independently selected from H, C₁-C₁₂ alkyl, C₆-C₁₄ aryl-C₁-C₆ alkyl, C₃-Cs cycloalkyl, or C₃-C₈ cycloalkyl-C₁-C₆ alkyl, wherein the C₆-C₁₄ aryl and the C₃-C₈ cycloalkyl are optionally substituted by one or more halogens, C₁-C₆ alkyl groups, C₁-C₆ alkoxyl groups, or C₁-C₆ alkyl-S- groups; or, R₁, R₂ and N connected thereto together form a N-containing five-membered ring; R₃ is selected from H, C₁-C₆ alkyl, or C₆-C₁₄ aryl-C₁-C₁₂ alkyl, wherein the C₆-C₁₄ aryl is optionally substituted by one or more halogens, C₁-C₆ alkyl groups, C₁-C₆ alkoxyl groups, or C₁-C₆ alkyl-S- groups; R₄ is selected from C₁-C₆ alkyl, C₁-C₆ alkoxyl, or C₁-C₆ alkyl-S-; at least one of R₁, R₂, or R₃ is not H; A is O; and n is 1.

4. The compound represented by Formula I, or the tautomer, optical isomer, nitrogen oxide, solvate, pharmaceutically acceptable salt or prodrug thereof according to any one of claims 1 to 3, wherein the C₁-C₁₂ aliphatic hydrocarbyl is selected from methyl, ethyl, n-propyl, isopropyl, n-butyl, isobutyl, tert-butyl, n-pentyl, isopentenyl, neopentyl, n-hexyl, vinyl, 1-propenyl, 2-propenyl, 1-methylvinyl, 1-butenyl, 1-ethylvinyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, or 1-hexynyl; the halogen is selected from F, Cl, Br, or I; the aryl is selected from phenyl or naphthyl; the C₃-Cs cycloalkyl is selected from cyclopropyl, cyclobutyl, cyclopentyl, or cyclohexyl; the N-containing four- to six-membered ring is selected from ethylenimine, pyrrolidine, piperidine, piperazine, morpholine, pyrrole, imidazole, pyrazole, thiazole, isothiazole, oxazole, isoxazole, pyridine, pyrazine, pyrimidine, or pyridazine; and the pharmaceutically acceptable salt is selected from hydrochlorides.

5. The compound represented by Formula I, or the tautomer, optical isomer, nitrogen oxide, solvate, pharmaceutically acceptable salt or prodrug thereof according to any one of claims 1 to 4, wherein a structure of Formula I comprises a structure of Formula II below:

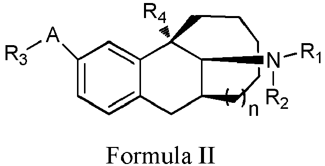

6. The compound represented by Formula I, or the tautomer, optical isomer, nitrogen oxide, solvate, pharmaceutically acceptable salt or prodrug thereof according to any one of claims 1 to 5, wherein the compound represented by Formula I comprises the following structures:

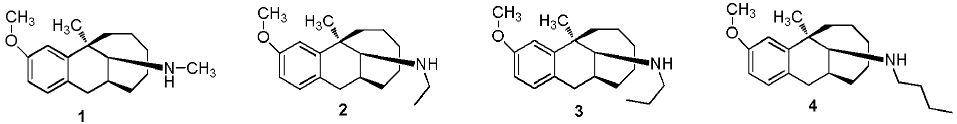

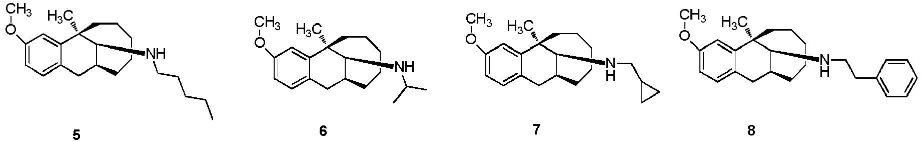

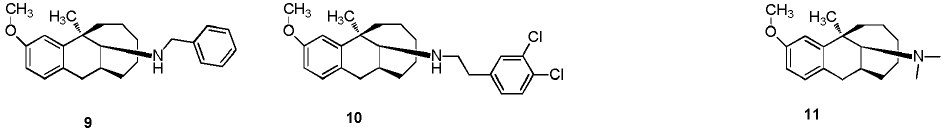

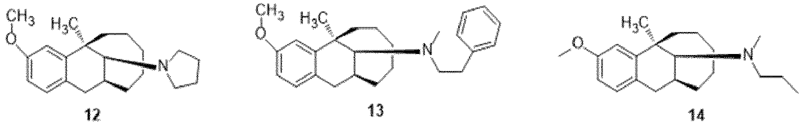

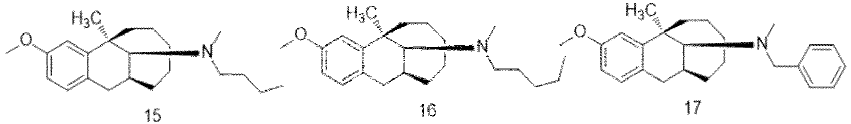

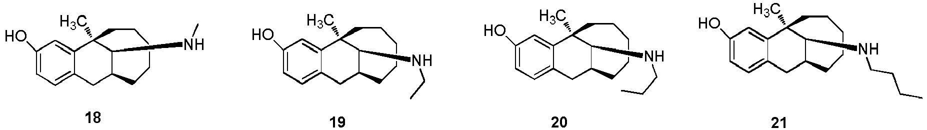

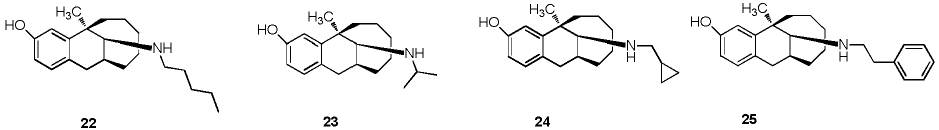

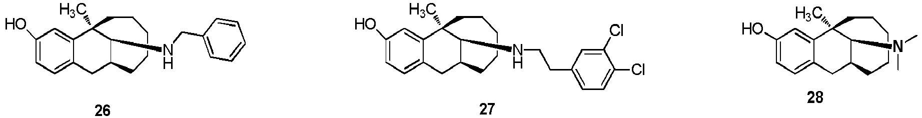

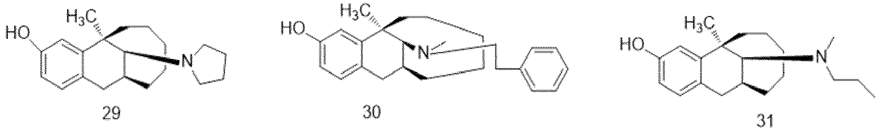

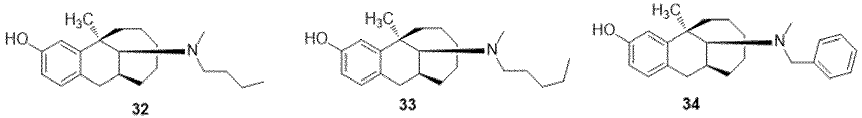

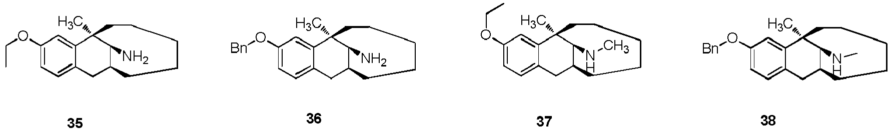

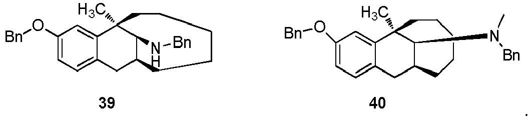

7. A preparation method of the compound represented by Formula I, or the tautomer, optical isomer, nitrogen oxide, solvate, pharmaceutically acceptable salt or prodrug thereof according to any one of claims 1 to 6, the preparation method being selected from the following synthesis schemes: 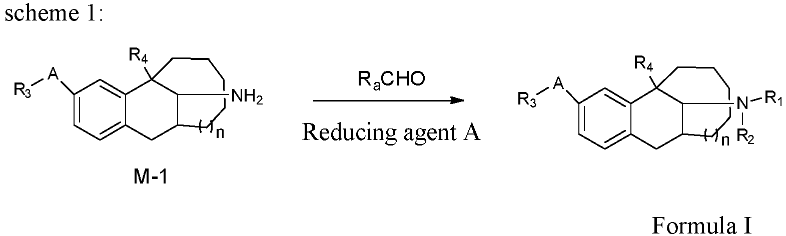 the scheme 1 comprising:
- compound M-1 reacting with an aldehyde RₐCHO to obtain an intermediate T-1; and
- obtaining the compound represented by Formula I through a reduction of the intermediate T-1,
wherein in the scheme 1, R₁, R₂, R₃, R₄, A, and n are those as defined in the Formula I, except that at least one of R₁ or R₂ is not H; RₐCHO is an aldehyde compound corresponding to substitutes R₁ and R₂ to be introduced in the target compound represented by Formula I, and allows a mono-substitution or di-substitution reaction with N, wherein when neither of R₁ nor R₂ is H, a disubstituted product is obtained correspondingly; and when one of R₁ and R₂ is H, a monosubstituted product is obtained correspondingly; 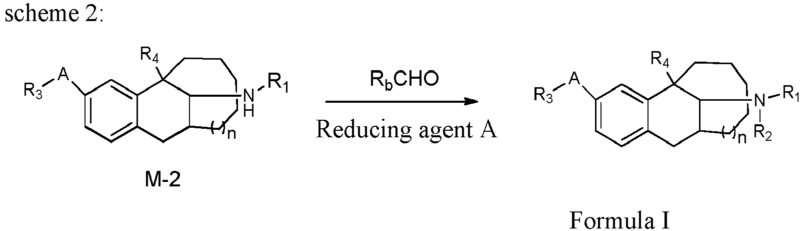 the scheme 2 comprising:
- compound M-2 reacting with an aldehyde R_{b}CHO to obtain an intermediate T-2; and
- obtaining the compound represented by Formula I through a reduction of the intermediate T-2,
wherein in the scheme 2, R₁, R₂, R₃, R₄, A, and n are those as defined in the Formula I, except that neither R₁ nor R₂ is H; R_{b}CHO is an aldehyde compound corresponding to R₂ to be introduced in the target compound represented by Formula I; 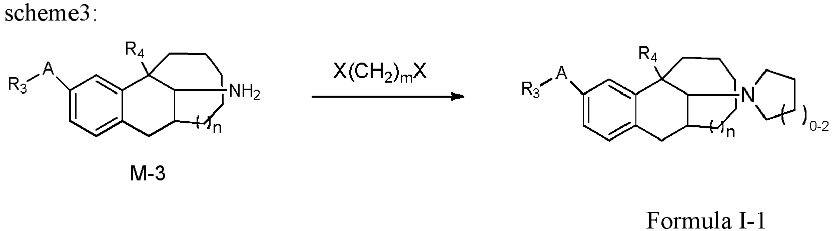 the scheme 3 comprising:
- compound M-3 reacting with compound X(CH₂)ₘX to obtain a compound represented by Formula I-1,
wherein in the scheme 3, R₃, R₄, A, and n are those as defined in the Formula I, m is from 3 to 5; and X is F, Cl, Br, or I; 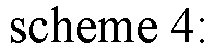 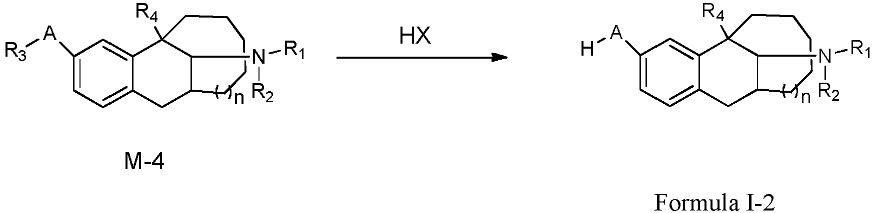 the scheme 4 comprising:
- compound M-4 reacting with HX to obtain a compound represented by Formula 1-2,
wherein in the scheme 4, R₁, R₂, R₃, R₄, A, and n are those as defined in the Formula I, except that R₃ is not H; and X is F, Cl, Br, or I; and 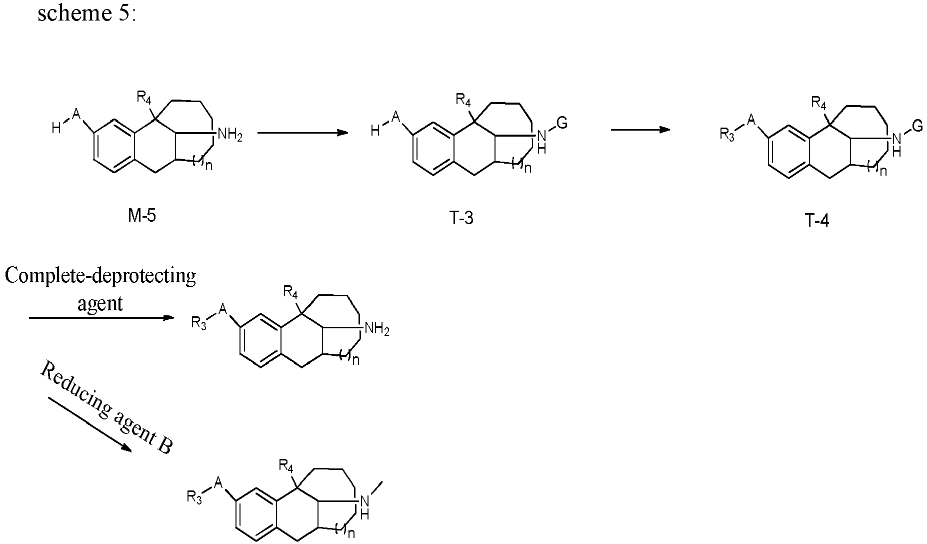 the scheme 5 comprising:
- compound M-5 reacting with an amino-protecting agent to obtain an amino-protected intermediate T-3;
- T-3 reacting with R₃X to obtain an intermediate T-4; and
- obtaining the compound represented by Formula I by completely deprotecting T-4, or obtaining a N-methylated product of the compound represented by Formula I from T-4 in presence of a reducing agent B,
wherein in the scheme 5, R₃, R₄, A, and n are those as defined in the Formula I, except that R₃ is not H; X is F, Cl, Br, or I; and G is an amino-protecting agent.

8. A pharmaceutical composition, comprising:
the compound represented by Formula I, or the tautomer, optical isomer, nitrogen oxide, solvate, pharmaceutically acceptable salt or prodrug thereof according to any one of claims 1 to 6; and optionally,
a second therapeutic agent, wherein the second therapeutic agent comprises MOR antagonists such as naloxone, naltrexone, tramadol, and samidorphan.

9. Use of the compound represented by Formula I, or the tautomer, optical isomer, nitrogen oxide, solvate, pharmaceutically acceptable salt or prodrug thereof according to any one of claims 1 to 6, or the pharmaceutical composition according to claim 8 in manufacture of a medicament for treating opioid receptor-related disorders, wherein the disorders comprise pain, hyperalgesia, and cardiovascular and cerebrovascular diseases; further, the disorders are pain, such as neuropathic pain or nociceptive pain; specific types of the pain comprise, but are not limited to, acute pain, chronic pain, postoperative pain, neuralgia-caused pain such as postherpetic neuralgia-caused pain or trigeminal neuralgia-caused pain, diabetic neuropathy-caused pain, toothache, arthritis- or osteoarthritis-associated pain, and pain associated with cancer or treatment thereof.

10. Use of the compound represented by Formula I, or the tautomer, optical isomer, nitrogen oxide, solvate, pharmaceutically acceptable salt or prodrug thereof according to any one of claims 1 to 6, or the pharmaceutical composition according to claim 8 in manufacture of a medicament for treating depression-related diseases and symptoms, wherein the depression-related diseases and symptoms comprise acute stress disorder, low mood adjustment disorder, Asperger's syndrome, attention deficit, bipolar disorder, borderline personality disorder, circulatory disorders, depression such as major depressive disorder (MDD) and treatment-resistant depression (TRD), dysthymic disorder, hyperactivity disorder, impulse control disorder, mixed mania, obsessive-compulsive personality disorder (OCD), paranoia, post-traumatic stress disorder, seasonal affective disorder, self-harm separation, sleep disorders, substance-induced emotional disorders.
